# FASCICULE DE BREVET EUROPEEN

(11) **EP 0 388 466 B1**
(45) Date de publication et mention de la délivrance du brevet: **19.04.1995**
(21) Numéro de dépôt: 89911558.8
(22) Date de dépôt: 27.09.1989
(51) Int. Cl.: C12N 15/57, C12P 21/02, C12N 9/64, C12Q 1/68, G01N 33/68, C12Q 1/37, A61K 38/48, C12P 21/08

(54) **ACIDE NUCLEIQUE CODANT POUR L'ENZYME DE CONVERSION DE L'ANGIOTENSINE (ECA) HUMAINE, ET SES APPLICATIONS, NOTAMMENT POUR LE DIAGNOSTIC $i(IN VITRO) DE L'HYPERTENSION ARTERIELLE**
FÜR DAS MENSCHLICHE ANGIOTENSION-UMFORMUNGSENZYM (ACE) KODIERENDE NUKLEINSÄURE UND IHRE ANWENDUNGEN, INSBESONDERE ZUR IN-VITRO-DIAGNOSE VON BLUTHOCHDRUCK
NUCLEIC ACID CODING FOR THE HUMAN ANGIOTENSINE CONVERSION ENZYME (ACE), AND ITS APPLICATIONS PARTICULARLY FOR THE $i(IN VITRO) DIAGNOSIS OF HIGH BLOOD PRESSURE

(30) Priorité: 27.09.1988 FR 8812620
(43) Date de publication de la demande: 26.09.1990
(73) Titulaire: INSTITUT NATIONAL DE LA SANTE ET DE LA RECHERCHE MEDICALE (INSERM), 75654 Paris Cédex 13 (FR)
(72) Inventeur: SOUBRIER, Florent, F-75016 Paris (FR); ALHENC-GELAS, François, F-75015 Paris (FR); HUBERT, Christine, F-92310 Sèvres (FR); CORVOL, Pierre, F-75007 Paris (FR)
(74) Mandataire: Gutmann, Ernest
(86) Numéro de dépôt international: FR8900496
(87) Numéro de publication internationale: WO9003435

(56) Documents cités:
- The Journal of Biological Chemistry, vol. 263, no. 23, 15 August 1988, (US), K.E. Bernsteim et al.: "The isolation of angiotensin - converting enzyme cDNA", pages 11021-11024, see the whole article.
- The Journal of Biological chemistry, vol. 260, no. 5, 10 March 1985, The American Soc. of Biological Chemists, Inc. (US) H.G. Bull et al.: "Purification of angiotensin-converting enzyme form rabbit lung and human plasma by affinity chromatrography", pages 2963-2972, see the whole article.
- Federation Proceedings (US) vol. 45, no. 6, 1986, T. Kreofsky et al.: "Purification of human lung antiotensin converting enzyme (ACE) and production of anti-catalytic monoclonal antibodies MoAb", see abstract no. 556.
- Biological Abstracts/RMM, accession no. 36018036, R.K. A. Allen et al.: "Monoclonal antibodies to human pulmonary angiotensin-converting enzyme", & Australian and New Zealand Journal of Medicine (Australia) 1988, vol. 18, no. 3,suppl. 2, p. 547, see the abstract.
- Chemical Abstracts, vol. 106, 1987, (Columbus, Ohio, US) see page 470, abstract no. 16946 & JP, A, 61186399 (FUJI PHARMACEUTICAL INDUSTRIES CO.; LTD) 20 August 1986
- Chemical Abstracts, vol. 108, 1988 (Columbus, Ohio, US) S.M. Danilov et al.: "Monoclonal antibody to human lung angiotensin-converting enzyme, see page 266, abstract no. 18303j & Biotechnol. App. Bioche. 1987, 9(4), 319-22.
- Chemical Abstracts, vol. 104, 1986, (Columbus, Ohio, US) H. Hiroshi et al.: "Pharmacological comparison of captopril and MK-422 by a new method for measuring activity of angiotension converting enzyme (ACE)" see page 7, abstract no. 179632Y & Jpn. Circ. J. 1985, 49(11), 1175-9.
- Chemical Abstracts, vol. 107, 1987 (Columbus, Ohio, US) C. Deluca-Flatherty et al.: "Hybridization and partical cDNA sequence analyses of bovine lung angiotensin I-converting enzyme", see page 169, abstract no. 148356u & Int. J. Pept. Protein Res. 1987, 26(6) 678-84.

## Description

L'invention concerne un acide nucléique codant pour l'enzyme de conversion de l'angiotensine (ECA) humaine ainsi que des vecteurs contenant cet acide nucléique et l'utilisation de ces derniers pour la production d'ECA humaine. L'invention concerne également les applications de cet acide nucléique, notamment dans le domaine de la conception de nouveaux inhibiteurs de l'ECA humaine.

L'ECA, ou peptidyl dipeptidase A (EC 3.4.15.1), ou encore kininase II, joue un rôle important dans la régulation de la pression artérielle, en hydrolysant l'angiotensine I (peptide inactif libéré après clivage de l'angiotensinogène par la rénine) en angiotensine II vasopressive jouant un rôle dans les mécanismes de l'hypertension artérielle (SKEGGS, L.T., et al (1986) J. Exp. Med., 103, 295-299).

L'inhibition de l'activité de l'ECA par l'EDTA et les chélateurs de métaux, indique qu'il s'agit d'une métallopeptidase, plus particulièrement d'une peptidase à zinc, capable d'hydrolyser, non seulement, l'angiotensine I, mais aussi la bradykinine (un peptide vasodilateur et natriurétique qu'elle transforme en un heptapeptide inactif), et de nombreux autres peptides à activité biologique (YANG, H.Y.T. et al (1970) Biochim. Biophys. Acta, 214, 374-376 ; ERDOS, E.G., et al (1987) Lab. Invest., 56, 345-348).

L'ECA est une peptidase largement distribuée dans l'organisme, que l'on retrouve par exemple sous forme d'enzyme membranaire à la surface des cellules endothéliales vasculaires, et des cellules épithéliales rénales, ainsi que sous forme d'enzyme circulant dans le plasma (ERDOS, et al (1987) sus-mentionné ; CARDWELL, P.R.B. et al, (1976) Science, 191, 1050-1051 ; RYAN, U.S. et al (1976) Tissue Cell, 8, 125-145).

Des méthodes de purification de l'ECA humaine ou animale ont déjà été décrites (notamment dans BULL H.G. et al, (1985)J. Biol. Chem., 260, 2963-2972 ; HOOPER, N.M. et al, (1987) Biochem. J., 247, 85-93).

Toutefois, la structure de l'ECA humaine n'est pas connue à l'heure actuelle. Seules quelques séquences peptidiques de l'ECA d'origine animale ont été publiées (BERNSTEIN, K.E. et al (1988), Kidney Int., 33, 652-655; HARRIS, R.B. et al (1985) J. Biol. Chem., 260, 2208-2211; IWATA, K. et al (1982) Biochem. Biophys. Res. Commun, 107, 1097-1103 ; IWATA, K. et al (1983) Arch. Biochem. Biophys., 227, 188-201 ; ST CLAIR, D.K. et al (1986) Biochem. Biophys. Res. Commun, 141, 968-972 ; SOFFER, RL. et al (1987) Clin. Exp. Hyp. A9, 229-234).

Quelques essais de clonage de l'ADN codant pour l'ECA animale ont été effectués à partir de deux organes riches en ECA, les reins et les poumons, mais aucun acide nucléique complet codant pour l'ECA animale n'a cependant été décrit ; seuls quelques fragments d'un tel acide nucléique ont été décrits (DELUCA-FLAHERTY, C. et al (1987) Int. J. Peptide Protein Res., 29, 678-684 ; BERNSTEIN K.E. et al, (1988 J. Biol. Chem., 263, 11021-11024)). Les quantités d'ARN messager (ARNm) codant pour l'ECA sont probablement trop faibles dans ces organes pour permettre le clonage d'un ADN complémentaire (ADNc) de cet ARNm. Aucun essai de clonage de l'ADN codant pour l'ECA humaine n'a été décrit jusqu'à maintenant.

Bien que le rôle physiologique de l'ECA dans les tissus extra-vasculaires ne soit pas encore connu, il est désormais bien établi que cette enzyme que l'on retrouve dans l'endothélium vasculaire et dans le plasma, joue un rôle essentiel dans l'homéostasie circulatoire par son action de clivage du dipeptide carboxy-terminal de l'angiotensine I, activant ainsi cette dernière en la transformant en angiotensine II qui est un peptide vasoconstricteur, stimulant la production d'aldostérone, et facilitant la transmission adrénergique.

Etant donné le rôle essentiel de l'angiotensine II dans le contrôle de la pression artérielle, une recherche active s'est développée sur la synthèse d'inhibiteurs de l'ECA. Le captopril a été le premier inhibiteur oral de l'ECA, suivi par de nombreux autres composés. Actuellement, les inhibiteurs de l'ECA occupent une place importante dans le traitement de l'hypertension artérielle. La structure complète de l'ECA n'étant pas connue, les inhibiteurs sus-mentionnés ont été conçus sur la base de la structure de protéases à zinc possédant des propriétés enzymatiques voisines de celles de l'ECA, notamment sur la base de la structure du site actif de la carboxypeptidase A (ONDETTI, M.A. et al (1977) Science, 196, 441-444).

Ainsi, en l'absence d'indication précise sur la structure de l'ECA, et plus particulièrement sur le ou les site(s) actif(s) de cette dernière, on conçoit facilement que les inhibiteurs de l'ECA sus-mentionnés synthétisés sur la base d'analogies avec la structure d'autres enzymes, peuvent être des molécules insuffisamment actives, ou non totalement spécifiques de l'ECA et susceptibles de posséder une action thérapeutique insuffisante, ou de créer des effets secondaires indésirables. Parmi ces effets indésirables, on distinguera notamment des phénomènes de toux, de troubles digestifs, d'éruptions cutanées qui peuvent être liés à l'inhibition d'autres systèmes enzymatiques par les inhibiteurs de l'ECA sus-mentionnés.

Un des buts de la présente invention est de permettre la conception de nouveaux inhibiteurs de l'ECA plus puissants et spécifiques de cette dernière que ne le sont les actuels inhibiteurs sus-mentionnés, permettant éventuellement une meilleure efficacité à dose moindre, et limitant les risques d'effets indésirables de ces nouveaux inhibiteurs.

La présente invention a pour objet Le clonage et le séquençage de l'acide nucléique codant pour l'ECA humaine ; ce travail a été réalisé à partir de deux banques d'ADN complémentaires d'ARNm provenant de cellules endothéliales de veines ombilicales humaines et de sondes nucléotidiques déduites à partir de séquences peptidiques obtenues par séquençage de fragments purifiés de l'ECA humaine. Les techniques utilisées pour le clonage et le séquençage de l'acide nucléique selon l'invention seront plus particulièrement décrites dans la description détaillée de l'invention.

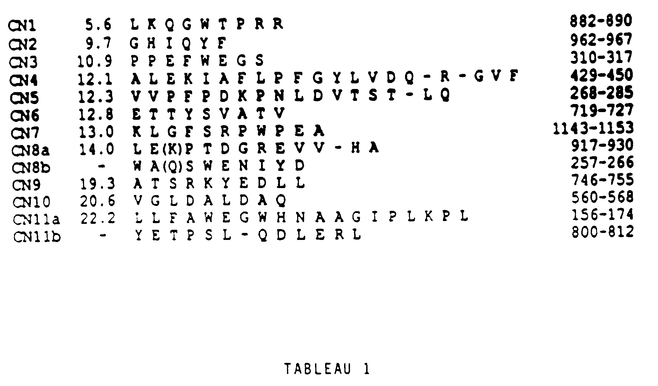

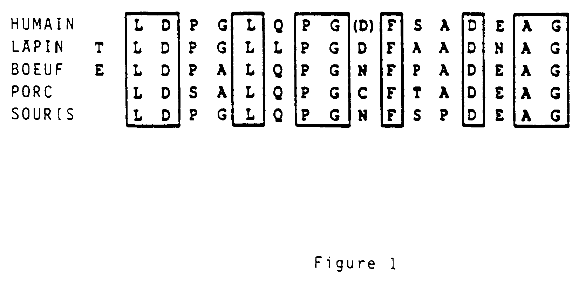

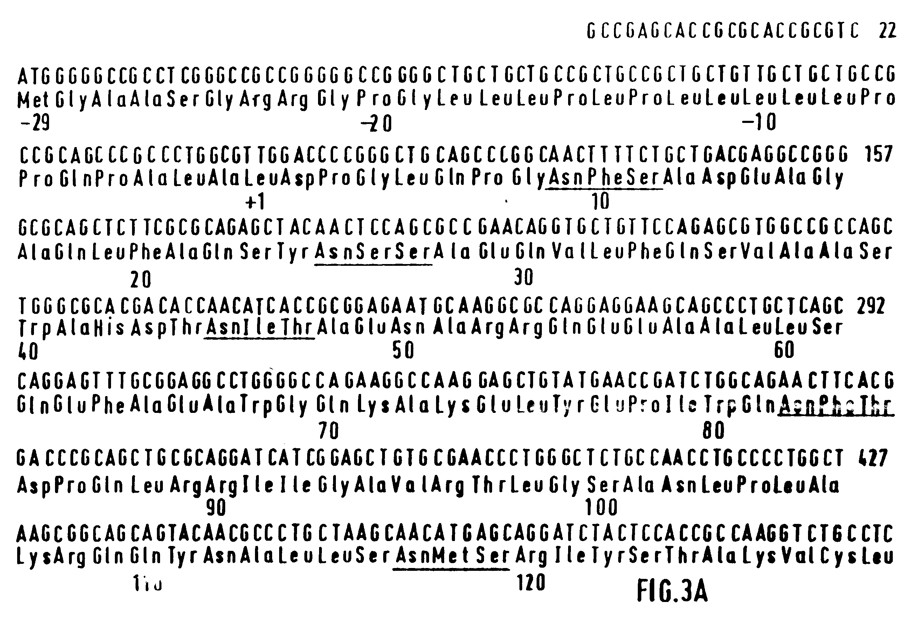

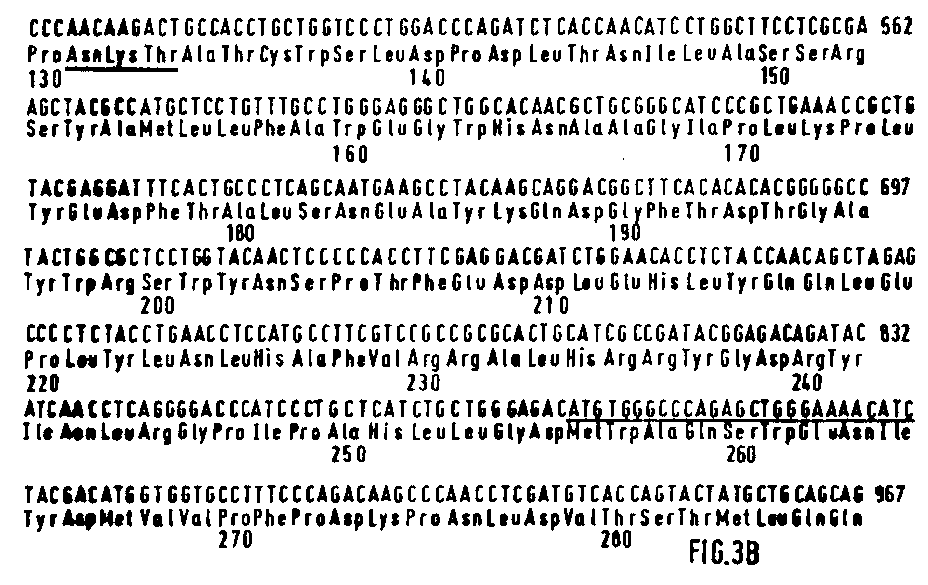

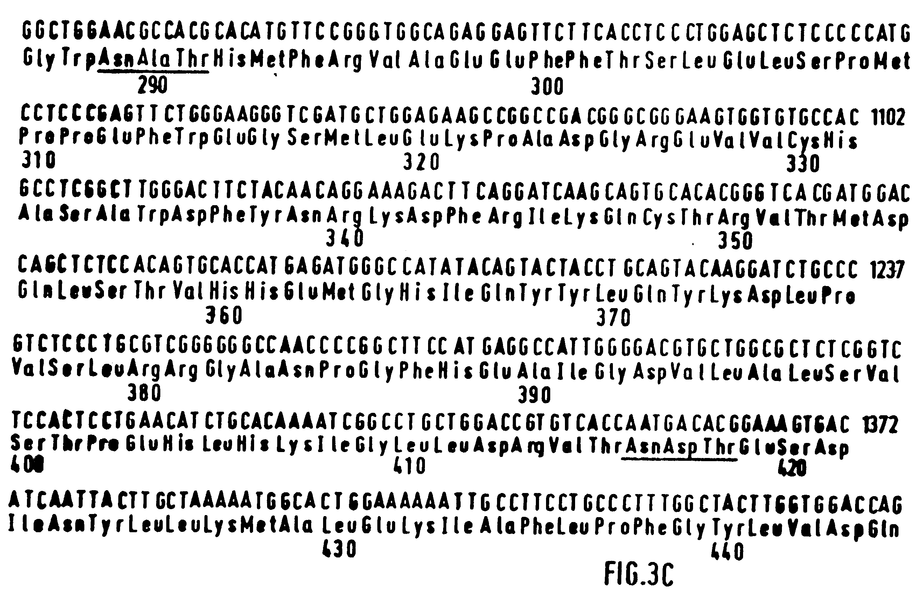

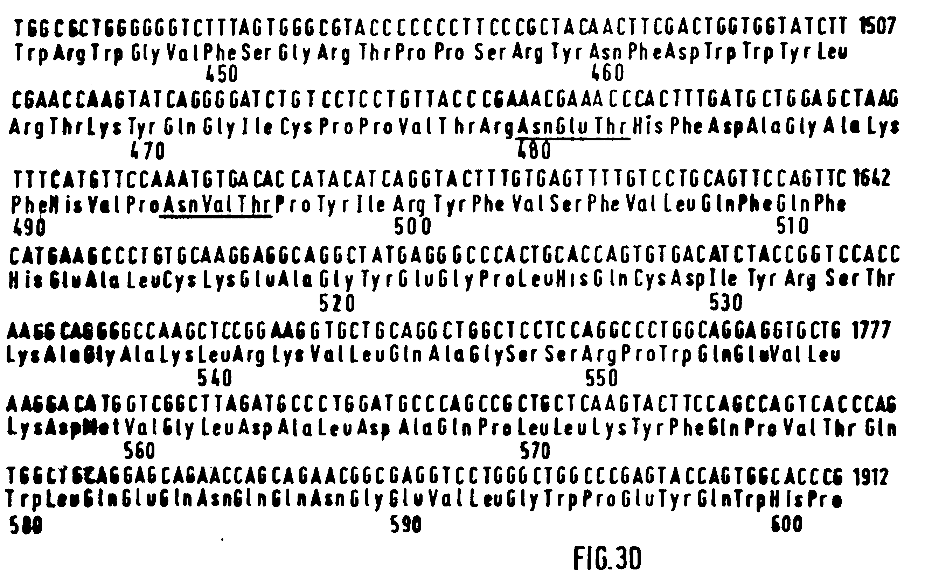

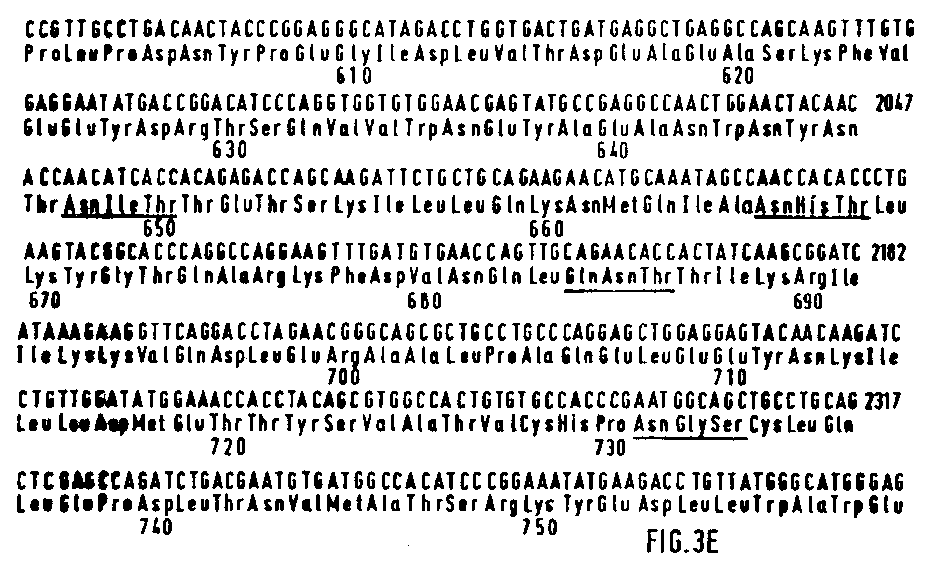

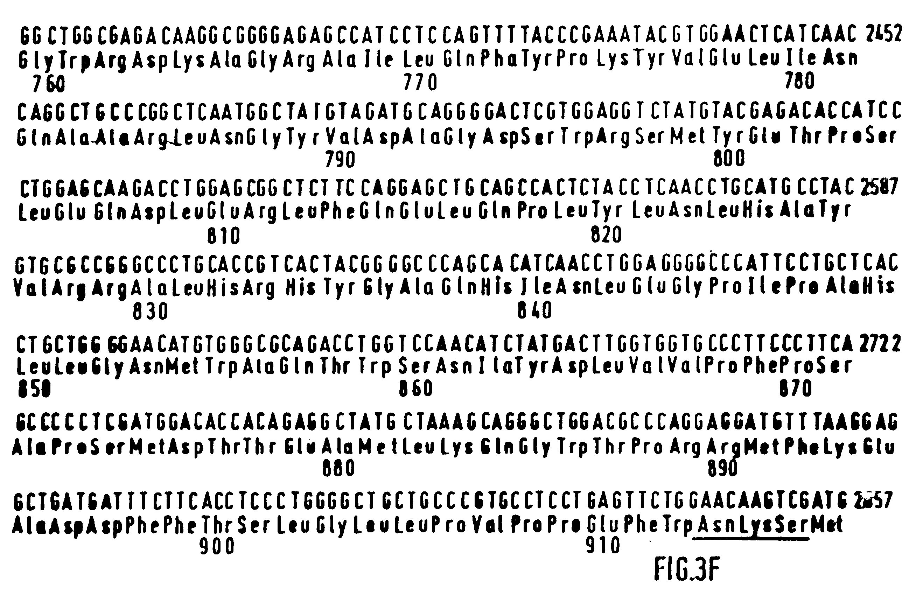

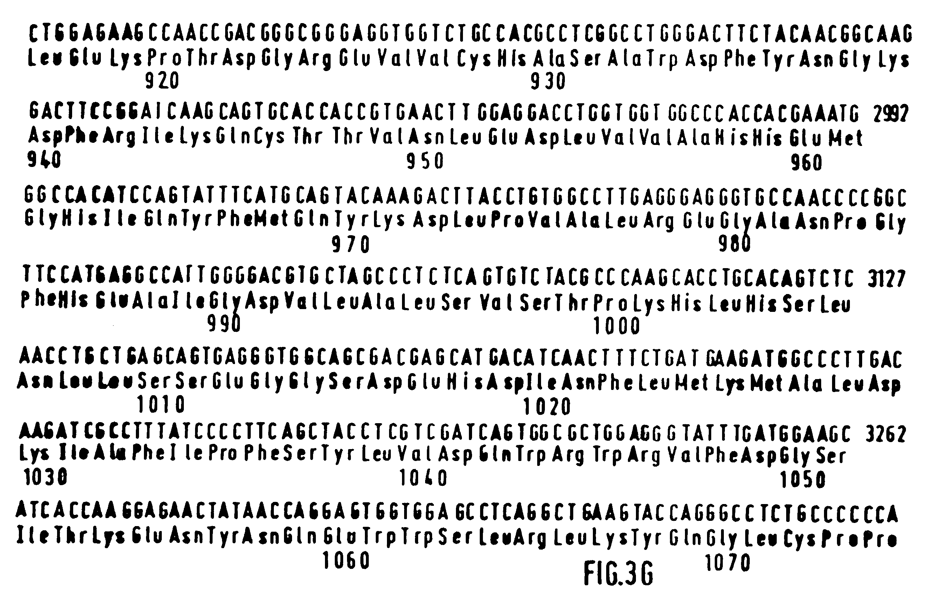

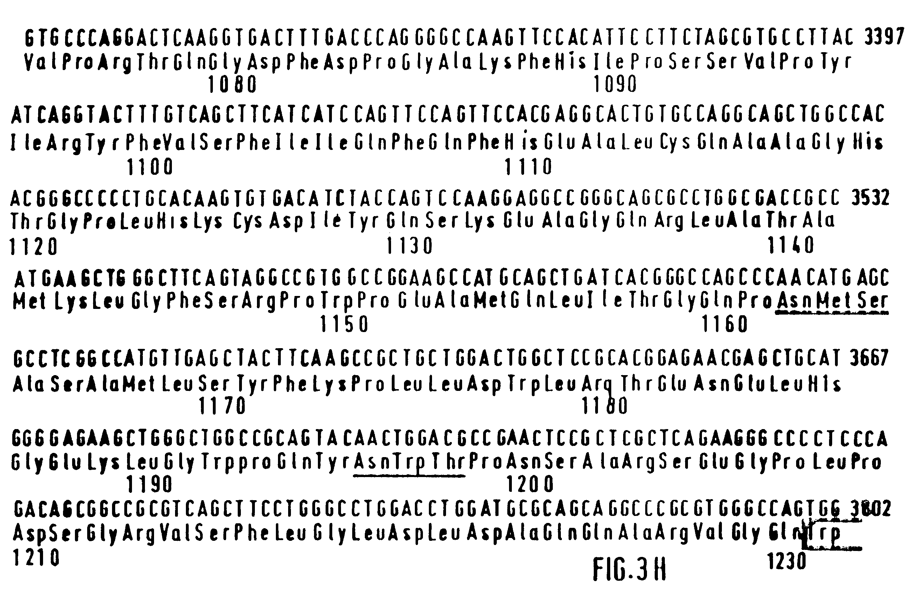

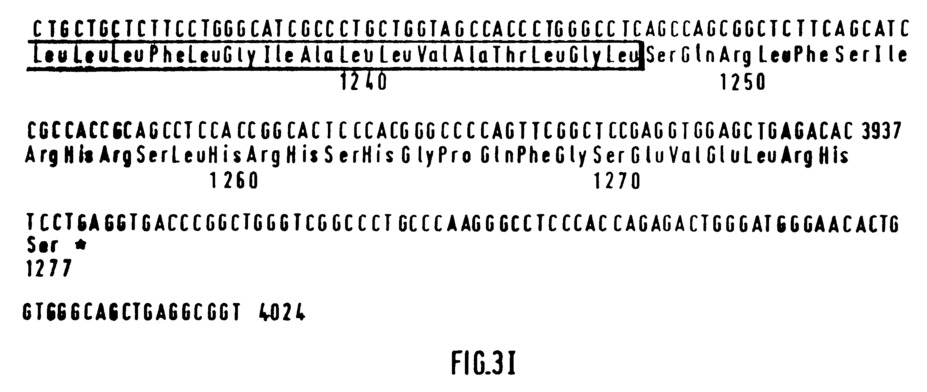

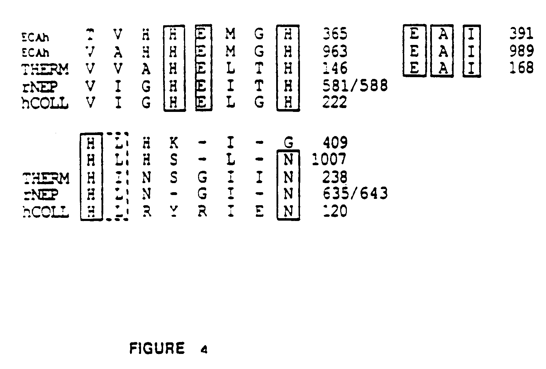

Il sera fait référence dans ce qui suit, au tableau I et aux figures dans lesquels :
- le tableau I représente les fragments peptidiques obtenus à partir de l'ECA humaine purifiée.
- la figure 1 représente la comparaison des séquences en acides aminés aminoterminales de l'ECA humaine, et des ECA provenant de lapins, de boeuf, de porc et de souris ;

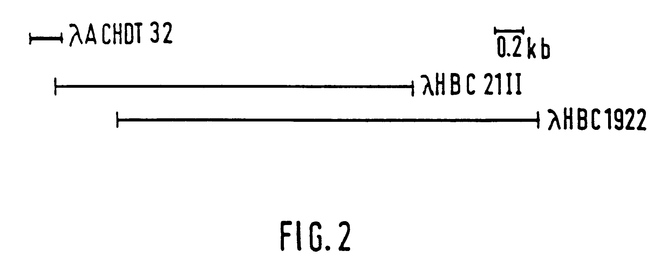

- la figure 2 représente les positions respectives des acides nucléiques des trois clones utilisés pour la détermination de l'acide nucléique codant pour l'ECA humaine;
- la figure 3 représente l'acide nucléique ainsi que le polypeptide, déduit de ce dernier, correspondant à l'ECA humaine ;
- la figure 4 représente les homologies entre certaines séquences peptidiques de l'ECA humaine (ECAh), de la thermolysine (THERM), de l'endopeptidase neutre de rat ou de lapin (rNEP), et de la collagénase de fibroblastes de peau humaine (COLLh) ;
Une étude approfondie de l'acide nucléique de l'invention, ainsi que du polypeptide déduit à partir de ce dernier et correspondant à l'ECA humaine, conduit aux observations suivantes :
- la phase de lecture ouverte de l'acide nucléique de la figure 3 est constituée d'une séquence d'ADN délimitée par les nucléotides correspondant aux positions 23 et 109 de la figure 1 codant pour un peptide signal de 29 acides aminés, et d'une séquence d'ADN délimitée par les nucléotides situés aux positions 110 à 3940 de la figure 3, et codant pour une protéine mature de 1277 acides aminés :
- l'acide nucléique de l'invention est caractérisé par une forte homologie interne (supérieure à 60 %) entre la séquence d'ADN délimitée par les nucléotides situés aux positions 700 et 1770, et celle délimitée par les nucléotides situés aux positions 2495 et 3565. Les polypeptides de 257 acides aminés codés respectivement par les deux séquences d'ADN susmentionnées présentent également une forte homologie entre eux (de l'ordre de 67,7 %). la plus forte homologie se situe au niveau des acides aminés des parties centrales des deux polypeptides sus-mentionnés ; par exemple le polypeptide délimité par les acides aminés situés aux positions 361 et 404, et celui délimité par les acides aminés situés aux positions 951 et 1002, présentent un pourcentage d'homologies de l'ordre de 89 %. Les deux polypeptides homologues comprennent chacun une séquence His-Glu-Met-Gly-His correspondant aux acides aminés situés aux positions 361 à 365 et 959 à 963 de la figure 3. Une étude comparative (représentée sur la figure 4) de cette dernière séquence peptidique de 5 acides aminés aminés, avec les sites actifs de plusieurs enzymes dont la thermolysine, suggère le fait que l'une au moins de ces deux séquences de 5 acides aminés, que l'on retrouve dans les deux parties homologues de l'ECA, pourrait constituer une partie du site actif de cette dernière ;
- le produit de la phase ouverte de lecture susmentionnée comprend 17 sites potentiels de glycosylation, dont la plupart sont groupés dans la région N-terminale de la molécule, et dans la région localisée à la jonction entre les deux domaines d'acides aminés homologues sus-mentionnés.

La présente invention concerne donc tout acide nucléique comprenant la séquence nucléotidique de la figure 3 codant pour l'ECA humaine.

L'invention concerne plus particulièrement tout acide nucléique comprenant la séquence d'ADN délimitée par les nucléotides situés aux positions 23 et 3940 de la figure 3, et codant pour la pré-ECA humaine de 1306 acides aminés ; les 29 premiers acides aminés du côté N-terminal représentent le peptide signal, et les 1277 acides aminés restant représentent l'ECA humaine nature.

L'invention concerne également tout acide nucléique comprenant la séquence d'ADN délimitée par les nucléotides situés aux positions 110 et 3940 de la figure 3, et codant pour l'ECA humaine mature.

L'invention a également pour objet tout acide nucléique issu de la séquence d'ADN de la figure 3 et codant pour un polypeptide susceptible de posséder des propriétés enzymatiques du type de celles de l'ECA humaine, notamment un polypeptide capable d'hydrolyser l'angiotensine I et/ou les kinines, notamment la bradykinine, ou tout acide nucléique ne se distinguant du précédent au niveau de sa séquence nucléotidique que par des substitutions de nucléotides n'entraînant la modification de la séquence en aminoacides du susdit polypeptide dans des conditions propres à lui faire perdre les susdites propriétés.

A ce titre, l'invention concerne plus particulièrement tout acide nucléique comprenant l'une ou l'autre des régions homologues de l'ECA susmentionnées.

Parmi les acides nucléiques conformes à l'invention, on citera notamment tout fragment d'ADN contenant :
- une séquence d'ADN s'étendant entre, d'une part, un nucléotide situé entre les positions 1 à 1177 et, d'autre part, un nucléotide situé entre les positions 3070 à 3940 ;
- une séquence d'ADN s'étendant entre, d'une part, un nucléotide situé entre les positions 110 à 1177, et d'autre part un nucléotide situé entre les positions 1276 à 1966 ;
- une séquence d'ADN s'étendant entre, d'une part, le nucléotide situé entre les positions 1967 à 2971, et, d'autre part, le nucléotide situé entre les positions 3070 à 3940.

L'invention concerne aussi les acides nucléiques dont les séquences nucléotidiques sont modifiées dans les limites autorisées par la dégénérescence du code génétique, dès lors que les polypeptides codés par ces acides nucléiques conservent soit une structure primaire identique, soit leurs propriétés enzymatiques ou immunologiques.

De telles modifications non limitatives conduisent par exemple à des acides nucléiques variants qui se distinguent des acides nucléiques ci-dessus :
- par addition et/ou
- suppression d'un ou de plusieurs nucléotides et/ou
- modification d'un ou de plusieurs nucléotides.

L'invention a plus particulièrement pour objet tout acide nucléique présentant la caractéristique de s'hybrider spécifiquement avec l'acide nucléique représenté sur la figure 3, dans les conditions définies ci-après :
- traitement de préhybridation du support (filtre de nitrocellulose ou membrane de nylon), sur lequel est fixé l'acide nucléique susceptible de s'hybrider avec celui de la figure 3, à 65°C pendant 6 heures avec une solution ayant la composition suivante : 4 x SSC, 10 x Denhardt,
- remplacement de la solution de pré-hybridation au contact du support par une solution tampon ayant la composition suivante : 4 x SSC, 1 x Denhardt, 25 mM NaPO₄ pH 7, 2 mM EDTA, 0,5 % SDS, 100 »g/ml d'ADN de sperme de saumon soniqué, comprenant l'acide nucléique de la figure 3 en tant que sonde, notamment de manière radioactive, et préalablement dénaturée par un traitement à 100°C pendant 3 minutes,
- incubation pendant 12 heures à 65°C,
- lavages successifs avec les solutions suivantes :
   . 2 x SSC, 1 x Denhardt, 0,5 % SDS, pendant 45 minutes à 65°C à quatre reprises,
   . 0,2 x SSC, 0,1 x SSC pendant 45 minutes à 65°C à deux reprises,
   . 0,1 x SSC, 0,1 % SDS pendant 45 minutes à 65°C.

Il est à rappeler que la composition de la solution de Denhardt est la suivante : 1 % ficoll, 1 % polyvinylpyrrolidone, 1 % BSA (albumine de sérum de boeuf), et que 1 x SSC est constituée de 0,15 M de NaCl et 0,015 M de citrate de sodium, pH 7.

L'invention concerne également tout acide nucléique présentant la caractéristique de s'hybrider spécifiquement avec l'acide nucléique de la figure 3 dans des conditions non stringentes reprenant les caractéristiques essentielles des conditions stringentes définies ci-dessus, sauf pour ce qui concerne la température qui est de 40°C dans les conditions non stringentes, et les lavages successifs qui, dans les conditions non stringentes, sont réalisés à l 'aide de 2 x SSC à 45°C pendant 15 minutes à 2 reprises.

L'invention vise également les sondes nucléotidiques susceptibles de s'hybrider avec les acides nucléiques décrits précédemment, ou leurs séquences complémentaires, ainsi qu'avec l'ARN messager codant pour l'ECA et avec le gène humain responsable de l'expression de l'ECA, dans les conditions d'hybridation définies ci-dessus.

Il va de soi que les conditions d'hybridation stringentes ou non stringentes, définies ci-dessus constituent des conditions préférées pour l'hybridation, mais ne sont nullement limitatives et peuvent être modifiées sans affecter pour autant les propriétés de reconnaissance et d'hybridation des sondes et des acides nucléiques sus-mentionnés.

Les conditions salines et de température, au cours de l'hybridation et du lavage des membranes, peuvent être modifiées dans le sens d'une plus grande ou d'une plus faible stringence sans que la détection de l'hybridation en soit affectée. Par exemple, il est possible d'ajouter de la formamide pour abaisser la température au cours de l'hybridation.
L'invention concerne également le polypeptide dont la séquence en acides aminés est représentée sur la figure 3, ainsi que tous les polypeptides susceptibles de posséder une activité enzymatique du type de celle de l'ECA et qui sont codés par les fragments d'ADN sus-mentionnés issus de l'acide nucléique de la figure 3.

Parmi les polypeptides sus-mentionnés, on distinguera notamment :
- le polypeptide s'étendant entre les acides aminés correspondant aux positions 1 et 619 de la figure 3 ;
- le polypeptide s'étendant entre les acides aminés correspondant aux positions 620 et 1229 ;
- le polypeptide s'étendant entre les acides aminés correspondant aux positions 350 et 395 ;
- le polypeptide s'étendant entre les acides aminés correspondant aux positions 948 et 993.

Les polypeptides précédents peuvent être modifiés dès lors qu'ils conservent les propriétés biochimiques ou immunologiques ou pharmacologiques définies précédemment.

Par exemple, et de façon non limitative, des polypeptides dans le cadre de l'invention peuvent se distinguer des polypeptides définis ci-dessus ;
- par addition et/ou
- suppression d'un ou plusieurs acides aminés et/ou
- modification d'un ou de plusieurs acides aminés, sous réserve que les propriétés biochimiques ou immunologiques ou pharmacologiques comme indiqué ci-dessus soient conservées.

On conçoit que l'homme de métier dispose de la possibilité d'identifier, voire de sélectionner ceux des polypeptides de séquences plus courtes qui entrent dans le champ de l'invention. A titre de l'un des moyens généraux lui permettant de procéder à cette identification, on mentionnera, par exemple, le traitement du polypeptide de la figure 3 avec une protéase clivant le polypeptide sus-mentionné dans un site peptidique choisi, soit dans une région N-terminale, soit dans une région C-terminale, suivi de la séparation du fragment N-terminal ou du fragment C-terminal du restant dudit polypeptide, ce "restant" étant alors testé pour son activité enzymatique vis-à-vis de l'angiotensine et/ou de la bradykinine. En cas de réponse positive, l'on aura alors établi que le fragment N-terminal ou C-terminal, ne jouait pas un rôle significatif, sinon essentiel pour la manifestation des propriétés enzymatiques dudit polypeptide. L'opération peut éventuellement être répétée pour autant que l'on dispose d'une protéase susceptible de reconnaître un autre site spécifique proche d'une extrémité N-terminale ou C-terminale du polypeptide restant. La perte par le polypeptide plus petit reconnu des propriétés enzymatiques du fragment plus long dont il était issu peut conduire à l'hypothèse que le dernier fragment séparé jouait un rôle significatif dans la manifestation des propriétés enzymatiques du polypeptide de la figure 3.

Une autre variante, plus simple que la précédente, de détection des régions de l'ECA essentielles à la manifestation des activités enzymatiques de cette dernière, peut être basée sur des traitements enzymatiques d'un acide nucléique codant pour l'ECA, avant l'incorporation de l'acide nucléique ainsi obtenu, et présumé coder pour un polypeptide possédant des activités enzymatiques du type de celle de l'ECA, dans le vecteur d'expression utilisé pour la mise en oeuvre d'un procédé de production dudit polypeptide dans l'hôte cellulaire approprié (procédé qui sera-plus particulièrement détaillé dans ce qui suit). Ce traitement enzymatique peut alors consister soit en un émondage des extrémités de l'acide nucléique initial (codant par exemple pour le polypeptide de la figure 3 en entier), par exemple par une enzyme exonucléolytique, telle que Bal31, soit par une ou plusieurs enzymes de restriction choisie pour leurs sites de reconnaissance respectifs (le cas échéant aménagés par mutagenèse ponctuelle dirigée) dans la séquence de l'acide nucléique initial, soit par l'addition d'un fragment d'ADN synthétique de jonction entre le site de coupure de l'enzyme de restriction et le début de la région à exprimer. L'acide nucléique tronqué obtenu peut alors être testé, après incorporation dans le vecteur choisi et transformation de l'hôte cellulaire avec le vecteur recombinant obtenu, pour sa capacité à exprimer un polypeptide tronqué correspondant, possédant encore les susdites propriétés enzymatiques -- ou au contraire ne les possédant plus, d'où la possibilité, comme dans la variante précédente, d'identifier au sein du polypeptide de la figure 3, les séquences jouant un rôle important, sinon essentiel dans la manifestation des propriétés enzymatiques de l'ECA.

L'invention a également pour objet tout acide nucléique recombinant contenant tout fragment d'ADN du type sus-indiqué codant pour la pré-ECA humaine, ou l'ECA humaine mature, ou encore pour tout polypeptide susceptible de posséder une activité enzymatique du type de celle de l'ECA, associé avec un promoteur et/ou un terminateur de transcription reconnu par les polymérases de la cellule hôte dans laquelle ledit acide nucléique recombinant est susceptible d'être introduit.

L'introduction dudit acide nucléique recombinant dans la cellule hôte, est avantageusement réalisée à l'aide de vecteurs, notamment du type plasmide, qui sont aptes à se répliquer dans ladite cellule hôte et à y permettre l'expression de la séquence codant pour l'enzyme.

Ledit acide nucléique recombinant peut également être introduit dans la cellule hôte à l'aide d'un vecteur viral (virus recombinant) capable d'infecter ladite cellule hôte et d'y permettre l'expression du polypeptide codé par un acide nucléique selon l'invention, ce dernier étant sous le contrôle d'un promoteur viral actif dans la cellule hôte.

L'invention concerne donc un procédé de production de l'ECA humaine, ou des polypeptides sus-mentionnés issus de l'ECA, qui comprend la transformation des cellules hôtes au moyen des vecteurs sus-indiqués, la mise en culture des cellules hôtes transformées dans un milieu approprié et la récupération desdits polypeptides soit directement à partir du milieu de culture, lorsque ces derniers y sont secrétés (notamment dans le cas où les polypeptides considérés sont précédés d'une séquence signal lors de leur synthèse dans la cellule hôte), soit après lyse de la paroi de la cellule hôte dans le cas où les polypeptides ne seraient pas secrétés hors de cette dernière.

Le procédé sus-mentionné comprend avantageusement une étape finale de purification, par exemple selon les techniques de chromatographie hybrophobe et d'affinité en faisant appel à un inhibiteur de toute ou partie de l'ECA fixé sur la colonne (cf. description détaillée qui suit).

A ce titre, l'invention a plus particulièrement pour objet une composition contenant de l'ECA humaine pure, exempte de contaminants notamment de nature protéique.

Les cellules hôtes utilisées pour la mise en oeuvre du procédé sus-mentionné peuvent être des cellules procaryotes, notamment des cellules de E. coli, ou, d'une manière plus avantageuse, des cellules eucaryotes, qui permettent, notamment, d'obtenir des protéines sous leur forme glycosylée et mature (levures, cellules CHO, ou cellules d'insectes infectées par le baculovirus).

Ce procédé décrit ci-dessus est également réalisable par transfection des cellules hôtes avec des vecteurs d'expression ayant intégré un gène modifié de l'ECA. Soit qu'il s'agisse de parties tronquées de l'ADN complémentaire de l'ARN messager endothélial de l'ECA, comprenant par exemple un seul des deux domaines homologues, ou bien amputé de l'extrémité 3' codant pour le segment hydrophobe d'insertion membranaire. La transfection des cellules hôtes est également réalisable avec des vecteurs comprenant une forme mutée de l'ECA, ou de ses fragments, les mutations portant en particulier sur les bases codant pour les acides aminés impliqués dans l'activité enzymatique décrits ci-dessus.

Il est particulièrement avantageux en vue de la cristallisation de l'enzyme recombinante de muter un ou plusieurs sites potentiels de N glycosylation, c'est-à-dire les codons correspondants aux asparagine des séquences asparagine-X-Threonine et asparagine-X-serine de la figure 3 (X représentant tout acide aminé compris entre une asparagine et une Thréonine ou une sérine de la figure 3).

L'invention vise également un procédé de préparation des nouveaux polypeptides sus-mentionnés par synthèse qui comprend soit l'addition étape par étape des résidus peptidiques choisis, avec l'addition ou l'élimination de groupes protecteurs quelconques des fonctions amino et carboxyle, ou addition de résidus peptidiques choisis afin de produire des fragments, suivie d'une condensation desdits fragments en une séquence en acides aminés appropriée, avec addition ou élimination des groupes protecteurs choisis.

L'invention se rapporte également à des anticorps spécifiques dirigés contre les polypeptides ci-dessus. En particulier, l'invention vise des anticorps monoclonaux dirigés contre les séquences peptidiques paraissant impliquées dans au moins une des activités enzymatiques de l'ECA.

De tels anticorps monoclonaux peuvent être produits par la technique des hybridomes dont le principe général est rappelé ci-après.

Dans un premier temps, on inocule un des polypeptides ci-dessus à un animal choisi, dont les lymphocytes B sont alors capables de produire des anticorps contre ce polypeptide. Ces lymphocytes producteurs d'anticorps sont ensuite fusionnés avec des cellules myélomateuses "immortelles" pour donner lieu à des hybridomes. A partir du mélange hétérogène des cellules ainsi obtenu, on réalise alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis des polypeptides de l'invention pourront être testés par exemple sur colonne d'affinité.

Parmi les polypeptides utilisés pour la fabrication des anticorps monoclonaux sus-mentionnés, on mentionnera notamment ceux délimités par les acides aminés situés aux positions 350 et 395, ou 948 et 993 de la figure 3.

L'invention concerne également une méthode de dépistage, ou de dosage, in vitro d'une ECA, et plus particulièrement de l'ECA humaine, ou d'un produit dérivé de l'ECA tel que les polypeptides sus-mentionnés ayant in vivo les propriétés de l'ECA, dans un prélèvement biologique susceptible de les contenir. Une telle méthode de dépistage selon l'invention, peut être réalisée soit à l'aide des anticorps monoclonaux susmentionnés, soit à l'aide des sondes nucléotidiques décrites ci-dessus.

Le prélèvement biologigue sus-mentionné est effectué soit dans des tissus fluides, tels que le sang, soit dans des organes, ce dernier type de prélèvement permettant notamment d'obtenir des coupes fines de tissus sur lesquelles les anticorps susmentionnés sont ultérieurement fixés.

La méthode de dosage selon l'invention, procédant par l'intermédiaire des anticorps sus-mentionnés comprend notamment les étapes suivantes :
- la mise en contact d'un anticorps reconnaissant spécifiquement l'ECA ou un polypeptide dérivé de l'ECA selon l'invention, avec le susdit prélèvement biologique dans des conditions permettant la production éventuelle d'un complexe immunologique formé entre l'ECA ou produit qui en est dérivé et l'anticorps susmentionné ;
- la détection à l'aide de tout moyen approprié du susdit complexe immunologique.

Avantageusement, les anticorps utilisés pour la mise en oeuvre d'un tel procédé sont marqués notamment de manière enzymatique ou radio-active.

Une telle méthode selon l'invention peut notamment être réalisée suivant la méthode ELISA (enzyme linked sorbent assay) qui comprend les étapes suivantes :
- fixation d'une quantité prédéterminée d'anticorps sur un support solide, notamment à la surface d'un puits d'une microplaque ;
- addition du prélèvement biologique (sous forme liquide) sur ledit support ;
- incubation pendant une temps suffisant pour permettre la réaction immunologique entre lesdits anticorps et l'ECA ou produit qui en est dérivé susmentionné ;
- élimination des parties non fixées du prélèvement biologique et lavage du support solide (en particulier des puits de la microplaque) ;
- addition d'une immunoglobuline marquée par une enzyme susceptible d'activer un substrat spécifique de cette enzyme,
- addition du substrat spécifique de l'activité enzymatique libérée lors de la réaction immunologique précédente ;
- détection, à l'aide de tout moyen approprié, de la dégradation du substrat par l'enzyme ; et
- corrélation de la quantité d'enzymes libérées à la concentration d'ECA humaine initialement présente dans le prélèvement biologique.

Selon un autre mode de réalisation de la méthode de dosage de l'invention, les anticorps sus-mentionnés ne sont pas marqués et la détection des complexes immunologiques formés entre les polypeptides et lesdits anticorps est réalisée à l'aide d'une immunoglobuline marquée reconnaissant lesdits complexes.

La méthode de dosage selon l'invention peut également être réalisée par une technique immunoenzymatique suivant un mécanisme de compétition entre les polypeptides susceptibles d'être contenus dans le prélèvement biologique, et des quantités prédéterminées de ces mêmes polypeptides, vis-à-vis des anticorps sus-mentionnés. Dans ce dernier cas, les polypeptides de l'invention en quantité prédéterminée sont avantageusement marqués à l'aide d'un marqueur enzymatique.

L'invention ne se limite nullement aux modes de réalisation décrits ci-dessus pour le dosage in vitro des polypeptides de l'invention, ce dosage pouvant être réalisé à l'aide de toute autre méthode immunologique appropriée.

L'invention concerne également une méthode de dépistage, ou de dosage, in vitro d'un acide nucléique codant pour toute ou partie de l'ECA, réalisée à partir d'un prélèvement biologique susceptible de contenir ledit acide nucléique, caractérisée en ce qu'elle comprend :
- la mise en contact d'une sonde nucléotidique décrite ci-dessus avec le susdit prélèvement biologique dans des conditions permettant la production éventuelle d'un complexe d'hybridation formé entre l'acide nucléique et ladite sonde ;
- la détection à l'aide de tout moyen approprié du susdit complexe d'hybridation.

Selon un mode de réalisation de l'invention, le prélèvement biologique sus-mentionné est, préalablement à la mise en oeuvre du dépistage, traité de manière à ce que les cellules qu'il contient soient lysées et, éventuellement, en ce que le matériel génomique contenu dans lesdites cellules soit fragmenté à l'aide d'enzymes de restriction du type EcoRI, BamHI etc..., ou que les ARN en soient isolés.

Avantageusement, les sondes nucléotidiques de l'invention sont marquées à l'aide d'un marqueur enzymatique ou radio-actif. Les ADN, ou ARN, issus du prélèvement biologique sont placés sur un support approprié, notamment sur un filtre de nitrocellulose ou autre, par exemple membrane de nylon, sur lequel sont ensuite additionnées les sondes sus-mentionnées.

Selon un autre mode avantageux de réalisation du procédé sus-mentionné de l'invention, des coupes histologiques sont réalisées à partir du susdit prélèvement biologique et les sondes nucléotidiques de l'invention sont mises en contact direct avec des coupes histologiques pour la détection des acides nucléiques de l'invention par hybridation in situ.

L'invention a également pour objet l'application des méthodes de dépistage, ou de dosage, susmentionnées au diagnostic in vitro de pathologies telles que l'hypertension, la sarcoïdose et autres maladies granulomateuses, les dysfonctionnements thyroïdiens et d'une manière générale toutes maladies corrélées à des teneurs dans l'organisme (dans le plasma ou d'autres tissus) en séquences d'acides aminés de l'invention, situées à l'extérieur du domaine délimité par les valeurs extrêmes correspondant généralement à l'état physiologique d'un individu sain.

Les méthodes de dosage in vitro de l'invention procédant à l'aide des anticorps sus-mentionnés permettent également le suivi dans l'organisme de la concentration des inhibiteurs de l'ECA par mesure de la quantité d'anticorps n'ayant pu se fixer sur le, ou les , site(s) actif(s) de l'ECA qui sont masqués par l'inhibiteur. Les méthodes de dosage de l'invention sont donc particulièrement avantageuses dans le cadre de la surveillance des traitements de patients par des inhibiteurs de l'ECA.

L'invention a également pour objet des nécessaires ou kits pour la mise en oeuvre des méthodes de dépistage, ou de dosage, in vitro sus-mentionnés.

A titre d'exemple, de tels kits comprennent notamment:
- une quantité déterminée d'un des anticorps monoclonaux sus-mentionnés susceptible de donner lieu à une réaction immunologique spécifique avec l'ECA ou avec un des polypeptides dérivés de l'ECA selon l'invention ;
- et/ou une quantité déterminée d'ECA ou un polypeptide susceptible de donner lieu à une réaction immunologique avec les anticorps sus-mentionnés ;
- avantageusement, un milieu approprié à la formation d'une réaction immunologique entre l'ECA ou les polypeptides de l'invention et les anticorps susmentionnés ;
- avantageusement, des réactifs permettant la détection des complexes immunologiques produits lors de la réaction immunologique sus-mentionnée.

Dans le cadre de la mise en oeuvre d'une méthode de dépistage in vitro utilisant des sondes nucléotidiques, les kits utilisés comprennent par exemple :
- une quantité déterminée d'une des sondes nucléotidiques sus-mentionnées susceptible de donner lieu à une réaction d'hybridation avec un des acides nucléiques sus-mentionnés codant pour l'ECA ou un polypeptide dérivé de l'ECA selon l'invention ;
- avantageusement, des réactifs permettant la détection des complexes d'hybridation produits lors de la réaction d'hybridation sus-mentionnée.

L'invention concerne également l'utilisation du polypeptide de la figure 3 ou de tout fragment peptidique approprié issu de ce dernier, pour la conception de nouveaux inhibiteurs de l'ECA humaine, plus puissants et/ou spécifiques de cette dernière que ne le sont les actuels inhibiteurs de l'ECA.

L'invention a également pour objet une méthode de détection ou de dosage, d'un inhibiteur de l'ECA, ou de quantification de son pouvoir inhibiteur, qui comprend la mise en contact du polypeptide de la figure 3, ou de tout fragment peptidique issu de de ce dernier et possédant une activité enzymatique du type de celle de l'ECA, avec ledit inhibiteur, et la détermination du coefficient d'inhibition de l'enzyme par l'inhibiteur, notamment par mesure de l'éventuelle activité enzymatique résiduelle sur un substrat approprié de l'ECA.

L'invention concerne également l'utilisation du polypeptide de la figure 3, ou de tout fragment de ce dernier capable d'hydrolyser les kinines, notamment la bradykinine, dans le traitement des maladies inflammatoires, ou infectieuses, de la pancréatite aigue, et plus généralement de maladies où une libération de kinines dans l'organisme pourrait jouer un rôle pathogène.

A ce titre, l'invention concerne plus particulièrement des compositions pharmaceutiques pour le traitement des maladies indiquées ci-dessus, caractérisée par l'association de tout ou partie du polypeptide de la figure 3, capable d'hydrolyser les kinines, notamment la bradykinine, avec un véhicule pharmaceutiquement acceptable.

La présente invention a également pour objet l'utilisation des sondes nucléotidiques de l'invention, capables de s'hybrider avec le gène responsable de l'expression de l'ECA humaine dans les conditions décrites ci-dessus, pour la détermination des différentes formes alléliques du gène sus-mentionné.

Les hypertensions artérielles (HTA) sont classées en deux grandes catégories selon leurs étiologies : les HTA secondaires et l'HTA essentielle. Les HTA secondaires regroupent toutes les formes d'HTA que la médecine peut rattacher de façon univoque à une pathologie identifiée. Il peut s'agir, notamment, d'une hypersécrétion hormonale d'origine tumorale (rénine ou aldostérone, catécholamines, par exemple) ou vasculaire (sténose d'une artère rénale provoquant une hypersécrétion de rénine). Ces formes d'HTA secondaires représentent environ 5 % du total des HTA. Sous le vocable d'HTA essentielle, sont regroupées toutes les formes d'HTA pour lesquelles aucune étiologie n'est aujourd'hui identifiable et qui constituent les 95 % restant des cas d'HTA.

La pathogénie de l'HTA essentielle n'est pas aujourd'hui connue mais de nombreuses études ont montré que des facteurs génétiques étaient impliqués dans le développement de l'HTA. Les études familiales ont montré qu'il existait une agrégation familiale des valeurs de pression artérielle et qu'une corrélation existe entre la pression artérielle des parents et des enfants naturels alors que cette corrélation n'existe pas avec les enfants adoptés. Les études menées sur des jumeaux mono ou dizygotes ont également confirmé l'héritabilité du niveau de pression artérielle. Enfin, l'analyse génétique de la transmission du niveau de pression artérielle, chez l'homme et dans les souches de rats génétiquement hypertendus, a montré que plusieurs gènes étaient impliqués. La transmission de ce caractère, variable entre les individus, qu'est le niveau de pression artérielle passe donc par la transmission de formes alléliques de gènes entre les parents et les enfants. Les gènes que l'on peut désigner "candidats" à la responsabilité de cette transmission sont, au premier chef, ceux impliqués dans les principaux systèmes de régulation de la pression artérielle, tels que les gènes, du système rénine-angiotensine, dont celui codant pour l'ECA.

La méthode de choix pour reconnaître, aujourd'hui, les formes alléliques d'un gène est d'identifier, pour ce gène, le polymorphisme de taille des fragments de restriction de ce gène (par taille des fragments de restriction on entend le nombre de paires de bases que comprennent lesdits fragments). Pour ce type d'expérience, les ADN de plusieurs individus sont isolés, clivés par une enzyme de restriction, transférés sur une membrane capable de les fixer de façon irréversible, et hybridés avec une sonde d'ADN marquée correspondant au gène étudié.

Cette méthode permet de distinguer les formes alléliques d'un même gène qui diffèrent entre elles d'un individu à l'autre :
- par leur carte de restriction, et/ou
- par la présence dans l'une des formes alléliques d'une insertion (fragment d'ADN supplémentaire dans l'allèle le moins fréquent), ou d'une délétion (fragment d'ADN manquant dans l'allèle le moins fréquent) cette insertion n'existant pas chez les autres formes alléliques.

Dans le premier cas sus-mentionné (différences fondées sur la carte de restriction, ou encore polymorphismes de sites de restriction), sous l'effet de la digestion par une enzyme de restriction donnée X, l'allèle possédant, au niveau d'une région précise du génome, un site de restriction reconnu par cette enzyme est coupé par ladite enzyme, et celui ne possédant pas un tel site, au niveau de ladite région, n'est pas clivé. A l'aide d'une sonde nucléotidique susceptible de s'hybrider au niveau de ladite région, on peut donc détecter deux fragments de restriction de tailles différentes selon que la région concernée a été clivée ou non. A chacune de ces tailles correspond une forme allélique.

Dans le second cas (présence d'une insertion, ou d'une délétion), les fragments d'ADN génomique correspondant aux deux formes alléliques sont clivés par l'enzyme de restriction X au niveau d'un site identique. On peut détecter, à l'aide d'une sonde telle que définie dans le paragraphe précédent, deux fragments de restriction de tailles différentes. A chacune de ces tailles correspond une forme allélique, la taille la plus élevée correspondant à la forme allélique comprenant l'insertion sus-mentionnée.

Dans les deux cas précédents et chaque fois que l'on détecte des fragments de restriction de tailles différentes (ou encore fragments de restriction polymorphes) dans un gène donné et d'un individu à l'autre sous l'effet de l'action d'une enzyme de restriction X, il sera fait référence au "polymorphisme X" de ce gène (par exemple "polymorphisme EcoRI" si l'enzyme de restriction X est l'enzyme EcoRI, ou polymorphisme TaqI si l'enzyme de restriction est TaqI).

L'étude d'un polymorphisme X d'un allèle choisi, dans une population donnée d'individus, présumés sains, permet de constater l'existence de ce polymorphisme dans une proportion déterminée des individus de cette population. La mesure de cette proportion conduit à ce que l'on appelle ci-après la "fréquence allélique de référence".

Si la fréquence d'un allèle choisi, mesuré suivant la même méthode au sein d'une population d'individus présentant une pathologie déterminée, est différente de la fréquence de référence, on pourra alors émettre l'hypothèse que l'allèle en question est lié à ladite pathologie.

De même, on pourra impliquer un allèle dans le développement de la maladie s'il existe une co-ségrégration entre la transmission de la maladie, dans les familles atteintes et informatives pour le polymorphisme, et la transmission de l'allèle permet d'impliquer cet allèle dans le développement de la maladie. (on dit d'une famille qu'elle est informative pour le polymorphisme si le parent atteint par la maladie est hétérozygote pour le site de restriction ce qui permet de suivre dans la descendance l'allèle responsable de la maladie).

La présente invention concerne les fragments de restriction polymorphes susceptibles de s'hybrider avec une sonde nucléotidique de l'invention, notamment dans les conditions décrites ci-dessus, et qui sont issus du clivage des différentes formes alléliques du gène codant pour l'ECA à l'aide d'enzymes de restriction déterminées.

L'invention concerne plus particulièrement les fragments de restriction polymorphes susceptibles de s'hybrider avec une sonde nucléotidique selon l'invention, elle-même susceptible de s'hybrider avec l'extrémité 5' de l'acide nucléique de la figure 3 et qui sont les suivants :
- les fragments de 5,8 kb et de 6 kb correspondant au polymorphisme TaqI,
- les fragments de 8,8 kb et de 9 kb correspondant au polymorphisme DraI,
- les fragments de 8,5 kb et de 9 kb correspondant au polymorphisme BglII,
- les fragments de 10,6 kb et de 11 kb correspondant au polymorphisme KpnI,
- les fragments de 8,4 kb et de 8,8 kb correspondant au polymorphisme HindIII,
- les fragmentsl de 4,2 kb et 3,0 kb d'une part, et de 4 kb et 2,7 kb d'autre part, correspondant au polymorphisme BglI,
- les fragments de 5,2 kb et de 5,7 kb correspondant au polymorphisme RsaI (avec présence ou non d'un fragment de 3,0 kb),
- les fragments de 4,3 kb d'une part, et de 2,2 et 2,5 kb d'autre part, correspondant au polymorphisme PvuII,
- les fragments de 3,5 kb et de 3 kb correspondant au polymorphismes XbaI,
- les fragments de 4,3 kb et de 2,7 kb correspondant au polymorphisme TaqI.

La différence de taille des fragments alléliques reconnus par les sondes de l'invention chez les différents individus, correspond à une insertion d'un fragment génomique d'environ 400 paires de base avec la marge d'erreur due à l'analyse de taille de ces fragments par électrophorèse sur gel d'agarose.

Cette insertion peut être détectée à l'aide de toute enzyme de restriction clivant l'acide nucléique sus-mentionné du moment que les fragments engendrés soient capables de s'hybrider au moins en partie, avec la sonde correspondante.

La présente invention concerne un procédé de dépistage, in vitro des polymorphismes du gène codant pour l'expression de l'ECA humaine, réalisé à partir d'échantillons biologiques, tels que le sang, susceptibles de contenir de l'ADN génomique, et qui ont été prélevés dans une population donnée d'individus ne présentant pas d'HTA (ou encore individus normotendus), ou d'autres maladies de l'endothélium vasculaire.

Les échantillons sus-mentionnés sont analysés suivant la méthode suivante comprenant :
- le traitement des acides nucléiques issus des échantillons biologiques sus-mentionnés à l'aide d'une enzyme de restriction déterminée dans des conditions permettant l'obtention de fragments de restriction issus du clivage desdits acides nucléiques au niveau de leurs sites de restriction reconnus par ladite enzyme,
- la mise en contact d'une sonde nucléotidique selon l'invention susceptible de s'hybrider avec les fragments sus-mentionnés dans des conditions permettant la production éventuelle de complexes d'hybridation entre ladite sonde et les fragments de restriction sus-mentionnés, notamment dans les conditions d'hybridation définies ci-dessus,
- la détection des susdits complexes d'hybridation,
- la mesure de la taille des éventuels fragments de restriction polymorphes engagés dans les complexes d'hybridation sus-mentionnés.

Parmi les sondes utilisées pour la mise en oeuvre d'un tel procédé, on citera notamment l'ADN complémentaire (ou ADNc) de l'acide nucléique décrit à la figure 3, ou, avantageusement, une fraction de cette ADNc susceptible de s'hybrider avec les différents fragments de restriction correspondant aux différentes formes alléliques du gène de l'ECA.

Avantageusement, la sonde utilisée dans le procédé sus-mentionné est marquée, notamment de manière radioactive ou non radioactive, sous forme notamment de sonde biotinylée, pour permettre la détection des différents fragments de restriction restant hybridés avec ladite sonde.

La mesure de la taille des différents fragments de restriction polymorphes engagés dans des complexes d'hybridation avec la sonde sus-mentionnée, peut être réalisée par toute méthode connue en soi par l'homme du métier ; la détermination de la taille de ces fragments de restriction est réalisée notamment par électrophorèse sur gel d'agarose et évaluation de la taille desdits fragments par rapport à celles de fragments témoins.

La comparaison des tailles des fragments de restriction mesurées chez les individus de la population étudiée, permet d'établir, au sein de cette population d'individus, les fréquences alléliques de référence.

L'invention concerne également la détection des différentes formes alléliques du gène codant pour l'ECA humaine, susceptibles d'être liées au développement chez un individu d'une HTA, ou autres maladies de l'endothélium vasculaire, notamment de l'athérosclérose. Cette détection est réalisée suivant la même méthode au sein d'une population d'individus présentant une pathologie sus-mentionnée. Si la fréquence d'un allèle mesurée au sein de cette population est différente de la fréquence de référence, on pourra alors émettre l'hypothèse que cet allèle est probablement lié à l'HTA.

De même, on pourra impliquer un allèle dans le développement de la maladie s'il existe, dans les familles atteintes par la maladie et informatives pour le polymorphisme, une coségrégation entre la transmission de certaines formes alléliques du gène de l'ECA et la transmission de l'HTA et/ou des maladies de l'endothélium, notamment de l'athérosclérose.

L'invention a également pour objet l'application des résultats de l'étude des polymorphismes du gène codant pour l'ECA humaine au diagnostic in vitro de l'éventuelle prédisposition génétique probable d'un individu à l'hypertension artérielle, ou à d'autres maladies de l'endothélium vasculaire, notamment de l'athérosclérose.

La méthode de diagnostic in vitro sus-mentionnée comprend la détection éventuelle de fragments de restriction polymorphes, issus du clivage par une enzyme de restriction déterminée d'allèles probablement liés au mécanisme de l'HTA, à l'aide de sondes nucléotidiques selon l'invention, susceptible de s'hybrider avec lesdits fragments de restriction polymorphes.

Les conditions dans lesquelles une telle méthode de diagnostic in vitro peut être mise en oeuvre sont celles décrites dans les articles concernant les techniques de détection des polymorphismes de longueur des fragments de restriction, plus connues encore sous l'abréviation RFLP (Restriction Fragment-length Polymorphism). De telles conditions sont plus particulièrement décrites dans l'article de GUSELLA J.F. 'Recombinant DNA techniques in the diagnosis of inherited disorders" paru dans Journal of Clinical Investigations (1986), 77, 1723-1726.

Selon un aspect avantageux de l'invention, outre le dépistage des polymorphismes du gène codant pour l'ECA humaine, la méthode de diagnostic in vitro susmentionnée comprend également le dépistage de polymorphismes d'un ou de plusieurs gènes codant pour des protéines différentes de l'ECA et impliquées dans le mécanisme de la régulation de la pression artérielle.

A ce titre, la méthode de diagnostic in vitro selon l'invention comprend le dépistage de polymorphismes du gène de l'ECA, et du gène de la rénine, et/ou du gène de la kallikréine, et/ou du gène de l'ANF.

Parmi les polymorphismes sus-mentionnés des gènes codant pour la rénine, la kallikréine, et l'ANF, on citera notamment ceux qui ont été plus particulièrement décrits dans la demande de brevet PCT publiée sous le numéro WO87/02709 déposée le 23 octobre 1987.

A l'aide de sondes appropriées, les auteurs de cette demande PCT ont découvert les fragments de restriction suivants :
- 5 kb et 9 kb correspondant au polymorphisme BglI du gène de la rénine,
- 0,9 kb correspondant au polymorphisme BglII du gène de la rénine,
- 20 kb et 24 kb correspondant au polymorphisme RsaI du gène de la rénine,
- 6,2 kb et 9 kb correspondant au polymorphisme HindIII du gène de la rénine,
- 9,8 kb et 11 kb correspondant au polymorphisme TaqI du gène de la rénine,
- 3,9 kb correspondant au polymorphisme EcoRI du gène de la rénine,
- 1,4 kb et 1,8 kb correspondant au polymorphisme BanII du gène ANF,
- 4,1 et 6,2 kb correspondant au polymorphisme BglI du gène ANF,
- 9,1 kb et 6,5 kb correspondant au polymorphisme BglII du gène ANF,
- 5,2 kb et 11,8 kb correspondant au polymorphisme EcoRI du gène ANF,
- 15 kb correspondant au polymorphisme EcoRI du gène de la kallikréine,
- 3,1 kb correspondant au polymorphisme PstI du gène de la kallikréine,
- 4,5 kb correspondant au polymorphisme StuI du gène de la kallikréine.

L'invention a également pour objet des kits pour la mise en oeuvre de la méthode de diagnostic in vitro définie ci-dessus.

A titre d'exemple, de tels kits de diagnostic comprennent :
- une quantité déterminée d'une ou de plusieurs enzymes de restriction,
- une quantité déterminée d'une sonde nucléotidique susceptible de reconnaître les fragments de restriction issus du clivage du gène codant pour l'ECA par l'(ou les ) enzyme(s) sus-mentionné(s),
- éventuellement une quantité déterminée d'une sonde susceptible de reconnaître les fragments de restriction issus du clivage du gène codant pour la rénine par l'(ou les) enzyme(s) sus-mentionnés(s),
- et/ou une quantité déterminée d'une sonde susceptible de reconnaître les fragments issus du clivage du gène codant pour la kallikréine par l'(ou les) enzyme(s) sus-mentionné(s),
- et/ou une quantité déterminée d'une sonde susceptible de reconnaître les fragments de restriction issus du clivage du gène codant pour l'ANF par l'(ou les) enzyme(s) sus-mentionné(s).

Les techniques classiques d'amplification génique, notamment celle décrite dans le brevet US n°4,683,202 déposé le 25/10/1985, sont utilisables pour détecter le polymorphisme du gène codant pour l'ECA en utilisant des amorces s'hybridant avec ce gène de part et d'autre de l'insertion d'environ 400 paires de base susmentionnée. Les fragments d'ADN ainsi amplifiés ont une taille différente selon que le fragment d'ADN génomique contient ou ne contient pas ladite insertion.

A ce titre, l'invention a plus particulièrement pour objet toute séquence nucléotidique d'environ 10 à 40 nucléotides issue de la séquence nucléotidique de la figure 3, ou sa séquence complémentaire, ou susceptible de s'hybrider avec une partie de la séquence de la figure 3 ou avec la séquence complémentaire de cette dernière, utilisable en tant qu'amorces pour l'amplification du gène codant pour l'ECA.

Des caractéristiques supplémentaires de l'invention apparaîtront encore au cours de la description qui suit du clonage de l'acide nucléique codant pour l'ECA humaine, ainsi que des sondes utilisées pour ce clonage, étant entendu que cette description ne saurait être interprétée comme tendant à restreindre la portée des revendications.

### I - PURIFICATION ET SEQUENCAGE DE L'ECA

L'ECA humaine sous sa forme complète membranaire a été purifiée à partir de microsomes rénaux. La fraction microsomale a été préparée à partir d'homogénats de reins de cadavres humains de la façon suivante : 600 g de cortex de rein ont été hachés, suspendus dans un tampon de phosphate de potassium 20 mM, pH 8, contenant 250 mM de saccharose et un mélange d'inhibiteurs de protéase (tétrathionate de sodium 5 mM, N-éthylmaléimide 10 mM, fluorure de phénylméthanesulfonyle 2 mM), et homogénéisés pendant 3 minutes. Les débris de tissu ont été éliminés par centrifugation a 5000 g pendant 20 minutes et la fraction particulaire a été sédimentée par centrifugation à 105000 g pendant 1 heure. Le culot a été resuspendu dans un tampon de phosphate de potassium 150 mM,; pH 8 (tampon I, 200 ml) et traité pendant 18 heures avec le détergent CHAPS 8 mM (Serva). Le surnageant, après centrifugation à 105000 g pendant 1 heure, a été dialysé contre le tampon I pendant 24 heures afin d'éliminer le CHAPS. Puis il a été déposé sur une colonne de phényl-Sépharose 4B (Pharmacia) et élué à un taux de 24 ml/heure ; 60 % de l'activité enzymatique a été retenue sur la colonne et éluée sous forme d'un pic unique après avoir appliqué un gradient linéaire de CHAPS (3-(3-cholamidopropyl)dimethylammonio-1-propanesulfonate) jusqu'à une concentration de 10 mM (800 ml). L'éluat a été enrichi avec du ZnSO₄ et du KCl a des concentrations finales de 0,1 mM et de 330 mM respectivement de manière à obtenir une liaison optimale avec l'inhibiteur, puis la purification a été complétée sur une colonne de lysinopril-Sépharose 4B comme il a été décrit dans BULL, H.G. et al, (1985), J. Biol. Chem., 260, 2963-2972.

La protéine ainsi isolée (1,2 mg) a été analysée, après réduction (2 mercaptoéthanol à 5 %) et dénaturation (ébullition pendant 3 minutes), par électrophorèse sur gel de polyacrylamide dans 6 % de gel contenant NaDodSO₄ (LAEMMLI, U.A., (1970), Nature, 227, 680-685).

L'enzyme purifiée hydrolyse l'angiotensine I, la bradykinine et plusieurs substrats synthétiques, et est inhibée par le captopril et d'autres inhibiteurs de l'ECA . la détermination de l'activité enzymatique a été réalisée à l'aide du substrat furanacryloyl-L-phenylalanyl-glycyl-glycine (FAPGG) dans les conditions décrites dans HOLMQUIST et al (1979), Anal. Biochem., 95, 540-548. Les paramètres de la réaction enzymatique déterminée à pH 7,5 sont de 136 »m pour Kₘ et 22100 min.⁻¹ pour Kcat ; un traitement à l'aide de bromure de cyanogène a été utilisé pour cliver l'enzyme au niveau des résidus méthionine générant ainsi des fragments pour le séquençage. L'ECA purifiée (0,5 mg) a été dissoute dans l'acide formique 70 % (0,2 ml) et le bromure de cyanogène (2,0 mg) a été ajouté. Après 16 heures de réaction à température ambiante dans le noir, la solution a été diluée 20 fois avec de l'eau et lyophilisée.

Les fragments peptidiques ont été isolés par HPLC en phase inverse en utilisant un système de gradient acide trifluoroacetique (TFA)-eau-acetonitrile (tampon A : 0,1 M de TFA dans l'eau ; tampon B : 0,1 % de TFA dans l'acetonitrile ; 1-100 % de B sur 32 minutes, taux d'élution 1 ml.min.⁻¹). Les fractions correspondant à des pics différents ont été séparées et lyophilisées, et séquencées à l'aide d'un séquenceur automatique. Les fragments peptidiques ainsi obtenus ont été regroupés sur le tableau I. La séquence amino-terminale de la protéine a été déterminée par la méthode d'Edman à l'aide d'un séquenceur automatique en utilisant la protéine ECA entière.

La séquence suivante de 16 acides aminés a été obtenue
NH₂-Leu-Asp-Pro-Gly-Leu-Gln-Pro-Gly-Asp-Phe-Ser-Ala-Asp-Glu-Ala-Gly-COOH

### II - CLONAGE DE L'ADN COMPLEMENTAIRE DE L'ARN MESSAGER

### A) Sonde nucléotidique

- à partir de la séquence peptidique
   Met-Trp-Ala-Met-Trp-Ala-Gln-Ser-Trp-Glu-Asn-Ile (conçue d'après la séquence CN8b du tableau I), 64 sondes nucléotidiques dénommées HACE6 ont été synthétisées à l'aide d'un synthétiseur d'ADN automatique en utilisant la méthode au phosphoramidite. 
Met-Trp-Ala-Lys-Ser-Trp-Glu-Asp-Ile-     3'
TAC-ACC-CGA-TTT-ACG-ACC-CTT-TTG-TA     5'
          G   G TG          C   A
- Marquage radio-actif : la sonde HACE6 a été marquée au [γP³²] ATP (activité spécifique : 5000 curies/mmole) à son extrémité 5' par la T4 polynucléotide kinase. L'activité spécifique de la sonde est de 5 x 10⁶ cpm/pmole.

### B) Criblage d'une banque d'ADN complémentaires construite à partie d'ARN de cellules endothéliales

Une banque d'ADNc de cellules endothéliales de veine ombilicale humaine amorcée avec de l'oligo(dT), construite dans le phage λgt11 (Clontech Laboratories Inc.), et constituée de 1,5 x 10⁶ recombinants indépendants, a été criblée en utilisant la sonde HACE6 marqué au P³². L'ADN des phages a été transféré sur filtre de nitrocellulose selon la méthode de Benton et Davis (réf. BENTON, W.D., et DAVIS R.W. (1977), Science (Wash), 196, 180-182). Les hybridations avec HACE6 ont été réalisées dans : 6 x SSC, 0, 1 % de NaDodSO₄, tampon NaPO₄ 50 mM à pH 6,8, 5 x Denhardt, 0,1 mg/ml d'ADN dénaturé de sperme de saumon à 50°C. Le lavage final des filtres a été effectué deux fois dans 2 x SSC, 0,1 % SDS, à 55°C pendant 15 minutes.

Dans ces conditions deux différents types de clones hybridant fortement avec la sonde HACE6 ont été obtenus. Il s'agit des clones λHEC1922 et λHEC2111. Les fragments d'ADN insérés dans les phages ont été isolés après coupure des phages par l'enzyme de restriction EcoRI, et insérés dans les deux sens dans le plasmide bluescript (Stratagene), au niveau du site EcoRI de ce dernier. A partir de ces plasmides doubles-brins des matrices mono-brins ont été obtenues en réinfectant des cultures bactériennes portant ces plasmides à l'aide d'un phage helper KO7.

La détermination de la séquence nucléotidique de ces clones a été effectué par la méthode de Sanger (SANGER, F. et al (1977), Proc. Natl. Acad. Sci., USA, 74, 5463-5467) en utilisant soit l'ADN polymérase de klenow, soit l'ADN polymérase de T7 modifiée (Sequenase, US Biochemical). Quelques régions ont été séquencées en utilisant à la place de la déoxyguanosine tri-phosphate (dGTP), le déoxyinosine tri-phosphate (dITP) ou le deaza-déoxyguanosine tri-phosphate (7-deaza dGTP). L'électrophorèse des fragments marqués par le [S³⁵] dATP a été pratiquée sur gel d'uréepolyacrylamide. Les séquences ont été déterminées en synthétisant des oligomères de proche en proche toutes les 350 paires de base environ qui ont servi d'amorces échelonnées le long de la séquence.

Les séquences nucléotidiques insérées dans ces deux phages sont chevauchantes sur une longueur de 2323 paires de base. La séquence peptidique déduite de ces clones contient l'ensemble des séquences peptidiques obtenues par purification de l'ECA humaine, et qui sont représentées sur le tableau I, à l'exception de la région amino-terminale.

De manière à obtenir l'ADN complémentaire correspondant à l'extrémité 5' de l'ARN messager codant pour l'ECA, une autre banque d'ADNc a été construite en utilisant 5 »g d'ARN poly(A) isolé à partir de cellules endothéliales de la veine ombilicale en culture tertiaire. Ces cellules ont été obtenues par dissociation à l'aide de collagénase comme il est décrit dans JAFFE et al (JAFFE J.M., et al (1973), J. Clin. Invest., 52, 2745-2756), et ont été cultivées en présence de 20 % de sérum foetal de veau, 100 »g/ml d'héparine et de 2ng/ml de FGF (facteur de croissance de fibroblaste) (GOSPORADOWICZ, D. et al (1983) J. Cell. Biol., 97, 1677-1685). L'ARN total a été extrait des cellules après 3 sous-cultures par la méthode de Chirgwin et al (CHIRGWIN, J.M. et al (1979) Biochemistry, 18, 5294-5299), et purifié sur cellulose oligo-d(T)7 (Pharmacia). La banque d'ADN complémentaire a été construite selon la méthode de Gubler et Hoffman (Gene (1983) 25, 263-269), en utilisant comme amorce un mélange de deux oligonucléotides. Le premier oligonucléotide est une amorce spécifique de l'ARNm de l'ECA, déterminé par la séquence des clones précédemment obtenus. Il s'agit d'un oligomère de 17 bases (CP5-21) complémentaire d'une séquence localisée près de l'extrémité 5' de l'ADNc de l'ECA (nucléotide 234 à 250 de la figure 3). La seconde amorce correspond à l'oligo-d(T)12-18 mers (Pharmacia). Les ADNc ont été traités et insérés dans le phage gGT10 coupé par l'enzyme EcoRI selon la méthode de Koenig et al (KOENIG, M. et al (1987), Cell, 50, 509-517).

Les phages recombinants ont été criblés à l'aide d'un fragment d'ADN génomigue humain de 300 paires de base isolé à partir d'une banque génomique clonée dans le phage CHARON4A et qui s'hybride avec CP5-21, et avec l'oligonucléotide de 44 bases obtenu à partir de la séquence amino-terminale déduite de l'ECA purifiée selon la méthode décrite ci-dessus.

Ce fragment de restriction (SacII-SacII) de 300 paires de base comprend la partie la plus 5' terminale de l'ARN messager de l'ECA ; il a été marqué avec une haute activité spécifique en utilisant la méthode de marquage décrite dans FEINBERG A.P. et al (1983) Anal. Biochem., 132, 6-13, et utilisé pour cribler 1,5 x 10⁶ clones dans des conditions de stringence élevée.

Parmi les clones positifs obtenus, le clone λCHDT32 a été sélectionné et séquencé selon la même méthode décrite pour les clones précédents. Il a ainsi été déterminé que ce clone contenait une insertion de 246 paires de base et qu'il démarrait 7 nucléotides en amont de l'amorce CP5-21 et qu'il s'arrêtait 25 nucléotides en amont de l'ATG d'initiation de la traduction.

L'insertion dans ce clone partage 60 paires de base avec le clone λHEC2111 et contient l'extrémité 5' de la séquence codant pour l'ECA.

La séquence nucléotidique de l'ADNc codant pour l'ECA obtenu par séquençage des trois clones précédents comprend donc 4024 nucléotides. L'extrémité 3' de cette séquence ne contient pas de signal de polyadénylation.

La phase ouverte de lecture partant du premier codon ATG jusqu'au codon stop TGA code pour 1306 acides aminés. La leucine amino-terminale déterminé par séquençage de la protéine est localisée après un peptide signal de 29 résidus.

Le clone λHEC1922 a été utilisé comme sonde pour étudier l'expression du gène codant pour l'ECA par la méthode dite "Northern-blot" selon le procédé de Thomas et al (THOMAS, P.S. et al (1983) Methods in Enzymology, N.Y. Academic Press, 100, 255-266). Un ARN messager d'environ 4,3 kb de cellules endothéliales de veine ombilicale en culture s'hybride avec la sonde susmentionnée. Dans le testicule, seul un ARN messager, plus court, de 3 kb a été détecté. Dans les reins, qui sont une source riche en ECA, aucune hybridation n'a été détectée dans les conditions utilisées en utilisant 20 »g d'ARN poly(A), ce qui laisse suggérer que la synthèse et les taux de turn-over de l'ECA sont faibles dans cet organe.

Une analyse par "Southern blot" de l'ADN humain dans des conditions stringentes a été réalisée avec un fragment EcoRI-BglII de 300 paires de bases localisé à l'extrémité 5' du clone λHEC2111. L'ADN humain a été isolé à partir de noyaux de cellules du placenta, par traitement avec la protéinase K suivi d'extractions par le mélange phénol-chloroforme. L'ADN (14 »g) a été digéré avec HindIII, séparé sur gel d'agarose a 0,7 % et analysé par hybridation selon la méthode "Southern Blot" (SOUTHERN, E.M. (1975) J. Mol. Biol, 98, 503-517).

Les fragments d'ADN ont été marqués selon la méthode décrite dans FEINBERG, A.P. et al (1983) anal. Biochem., 132, 6-13. Cette sonde s'hybride avec un fragment de restriction unique de 9,5 kb.

Des résultats similaires ont été obtenus en utilisant un fragment d'ADNc proche de l'extrémité 3'.

Ce résultat confirme la présence d'un seul gène.

La détermination de l'activité enzymatique de l'ECA codée par le gène ainsi isolé, ou de tout polypeptide actif selon l'invention et issu de cette dernière, peut être déterminée notamment par la méthode décrite par Holmquist et al (1979) précédemment cité.

Le dosage de cette ECA humaine peut être réalisé par des techniques radioimmunologiques (RIA) décrites par HIVADA K., et al (1987) Lung, 267, 27-35.

### III POLYMORPHISMES DU GENE DE L'ECA

L'ADN de plusieurs individus a été isolés à partir des cellules nucléées par lyse des globules rouges suivies d'une lyse des globules blancs, d'un traitement à la protéinase K et par extractions successives par le phénol et le chloroforme suivies d'une précipitation à l'isopropanol.

Une quantité déterminée d'ADN est ensuite digérée par une enzyme de restriction et migrée sur un gel d'agarose qui sépare les fragments d'ADN en fonction de leur taille. L'ADN est ensuite dénaturé par un traitement avec de la soude 0,5 N et transféré sur une membrane de nylon par buvardage pendant 12 heures. L'ADN est ensuite fixé à la membrane par passage au four pendant 2 heures à 80°C.

La membrane est ensuite pré-hybridée avec une solution 4 x SSC, 10 x Denhardt pendant au moins 6 heures à 65°C. La membrane est ensuite hybridée avec une solution tampon 4 x SSC, 1 x Denhardt, 25 mM NaPO₄ pH 7, 2 mM EDTA, 0,5 % SDS, 100 »g/ml d'ADN de sperme de saumon soniqué comprenant la sonde marquée dénaturée par la chaleur à 100°C pendant 3 minutes.

La sonde marquée est un mélange des fragments ADNc de l'ECA contenu dans les clones λHEC1922 et λHEC1922.

Les fragments d'ADN purifiés sont marqués à haute activité spécifique par la technique d'amorçage statistique utilisant le fragment de Klenow de la polymérase de E. coli et un déoxynucléotide marqué au P³² à haute activité spécifique.

Les membranes sont hybridées en présence de la sonde pendant 12 heures à 65°C.

Après l'hybridation, la membrane est lavée dans une solution 2 x SSC, 1 x Denhardt, 0,5 % SDS à 65°C pendant 45 minutes à quatre reprises, lavée ensuite dans une solution 0,2 x SSC, 0,1 x SSC pendant 45 minutes à 65°C à deux reprises, puis dans une solution 0,1 x SSC, 0,1 % SDS pendant 45 minutes à 65°C.

Les séquences ayant hybridé avec la sonde sont ensuite visualisées par autographie des membranes à -80°C en présence d'un écran intensificateur sur un film.

L'analyse de l'ADN de plusieurs individus avec les sondes ADNc de l'ECA ou des sondes équivalentes c'est-à-dire raccourcies à l'une des deux extrémités, ou allongées vers l'extrémité 3' ou 5' de la partie transcrite ou non transcrite du gène, ou des fragments d'ADN synthétiques qui gardent la propriété de s'hybrider avec les fragments de restriction susmentionnés permet de distinguer plusieurs types de fragments de restriction en fonction des coupures enzymatiques de l'ADN. Ces fragments sont les suivants:
- 5,8 kb ou 6 kb par digestion TaqI,
- 8,8 kb ou 9 kb par digestion DraI,
- 8,5 kb ou 9 kb par digestion DglII,
- 10,6 kb ou 11 kb par digestion KpnI,
- 8,4 kb ou 8,8 kb par digestion HindIII,
- 4,2 kb et 3,0 kb ou 4 kb et 2,7 kb par digestion BglI;
- 5,2 kb ou 5,7 kb par digestion RsaI, avec présence ou non d'une bande à 3,0 kb.
- 3,5 kb ou 3 kb par digestion XbaI,
- 4,3 kb ou 2,7 par digestion TaqI.

Par digestion par l'enzyme PvuII, on obtient deux profils ou pattern de restriction comportant la présence ou non d'une bande à 4,2 kb qui est associée à une bande plus faible de 2,2 et de 2,5 kb.

## Revendications

1. Acide nucléique caractérisé en ce qu'il comprend toute ou partie de la séquence nucléique de la figure 3, et en ce qu'il code pour un polypeptide capable d'hydrolyser l'angiotensine I et/ou les kinines, notamment la bradykinine, ou tout acide nucléique ne se distinguant du précédent au niveau de sa séquence nucléotidique que par des substitutions de nucléotides n'entraînant pas la modification de la séquence en aminoacides du susdit polypeptide dans des conditions propres à lui faire perdre les susdites propriétés.

2. Acide nucléique selon la revendication 1, caractérisé en ce qu'il comprend une séquence d'ADN délimitée par les nucléotides situés aux positions 110 et 3940 de la figure 3, codant pour l'ECA humaine mature.

3. Acide nucléique selon la revendication 2, caractérisé en ce que ladite séquence d'ADN codant pour l'ECA humaine mature est précédée par une séquence d'ADN délimitée par les nucléotides situés aux positions 23 et 109 de la figure 3, cette dernière codant pour un peptide signal de 29 acides aminés.

4. Acide nucléique selon la revendication 1, caractérisé en ce qu'il comprend une séquence d'ADN s'étendant entre, d'une part, le nucléotide situé entre les positions 1 à 1177 et, d'autre part, le nucléotide situé entre les positions 3070 à 3940 de la figure 3.

5. Acide nucléique selon la revendication 1, caractérisé en ce qu'il comprend une séquence d'ADN s'étendant entre, d'une part, le nucléotide situé entre les positions 110 à 1177 et, d'autre part, le nucléotide situé entre les positions 1276 à 1966 de la figure 3.

6. Acide nucléique selon la revendication 1, caractérisé en ce qu'il comprend une séquence d'ADN s'étendant entre, d'une part, le nucléotide situé entre les positions 1967 à 2971 et, d'autre part, le nucléotide situé entre les positions 3070 à 3940 de la figure 3.

7. Acide nucléique , caractérisé en ce qu'il comprend une séquence nucléotidique selon l'une quelconque des revendications 1 à 6 associée avec un promoteur sous le contrôle duquel la transcription de la ladite séquence est susceptible d'être d'effectuée et, le cas échéant, une séquence d'ADN codant pour des signa signaux de terminaison de la transcription.

8. Vecteur recombinant, notamment plasmide recombinant, ou virus recombinant, capable de transformer, ou d'infecter, une cellule hôte appropriée, notamment une cellule eucaryote, ledit vecteur contenant un fragment d'ADN selon l'une quelconque des revendications 1 à 7 sous le contrôle d'éléments de régulations permettant l'expression de ce fragment d'ADN dans la cellule hôte, notamment d'un promoteur reconnu par les polymérases de ladite cellule hôte.

9. Procédé de production de l'ECA humaine, mature ou non, ou d'un polypeptide issu de l'ECA humaine et capable d'hydrolyser l'angiotensine I et/ou les kinines, notamment la bradykinine, ce procédé comprenant la transformation de cellules hôtes au moyen du vecteur selon la revendication 8, la mise en culture des cellules hôtes transformées dans un milieu de culture approprié et la récupération de l'enzyme à partir de ces cellules ou du milieu de culture.

10. Polypeptide codé par l'acide nucléique selon la revendication 1, caractérisé en ce qu'il comprend toute ou partie de la séquence peptidique délimitée par les acides aminés situés aux positions -29 à 1277 de la figure 3, et en ce qu'il est capable d'hydrolyser l'angiotensine I et/ou les kinines, notamment la bradykinine.

11. Polypeptide selon la revendication 10, caractérisé par la séquence peptidique délimitée par les acides aminés situés aux positions 1 et 619 de la figure 3.

12. Polypeptide selon la revendication 10, caractérisé par la séquence peptidique délimitée par les acides aminés situés aux positions 620 et 1229 de la figure 3.

13. Polypeptide selon la revendication 10, caractérisé par la séquence peptidique délimitée par les acides aminés situés aux positions 350 et 395 de la figure 3.

14. Polypeptide selon la revendication 10, caractérisé par la séquence peptidique délimitée par les acides aminés situés aux positions 948 et 993 de la figure 3.

15. Anticorps notamment monoclonal, caractérisé en ce qu'il est dirigé contre un polypeptide selon l'une quelconque des revendications 10 à 14.

16. Sonde nucléotidique caractérisée en ce qu'elle est constituée par une séquence nucléotidique complémentaire de toute ou partie de l'acide nucléique de la revendication 1, ou toute sonde nucléotidique ne se distinguant de la précédente au niveau de séquence nucléotidique que par des substitutions de nucléotides n'entraînent pas la modification des propriétés d'hybridation de ladite sonde avec l'acide nucléique de la revendication 1.

17. Procédé d'hybridation d'une sonde nucléotidique selon la revendication 16 avec un acide nucléique selon l'une quelconque des revendications 1 à 6, caractérisé en ce qu'il comprend, dans les conditions d'hybridation stringentes, les étapes suivantes :
- traitement de préhybridation du support (filtre de nitrocellulose ou membrane de nylon) sur lequel est fixé l'acide nucléique selon l'une quelconque des revendications 1 à 6, à 65°C pendant 6 heures avec une solution ayant la composition suivante : 4 x SSC, 10 x Denhardt ;
- remplacement de la solution de pré-hybridation au contact du support par une solution tampon ayant la composition suivante : 4 x SSC, 1 x Denhardt, 25 mM NaPO₄ pH 7, 2 mM EDTA, 0,5 % SDS, 100 »g/ml de sperme de saumon soniqué, comprenant la sonde nucléotidique, marquée notamment de manière radioactive, et préalablement dénaturée par un traitement à 100°C pendant 3 minutes ;
- incubation pendant 12 heures à 65°C ;
- lavages successifs avec les solutions suivantes :
. 2 x SSC, 1 x Denhardt, 0,5 % de SDS pendant 45 minutes à 65°C à quatre reprises,
. 0,2 x SSC, 0,1 x SSC pendant 45 minutes à 65 °C à deux reprises,
. 0,1 x SSC, 0,1 % SDS pendant 45 minutes à 65°C.

18. Procédé d'hybridation d'une sonde nucléotidique selon la revendication 16 avec un acide nucléique selon l'une quelconque des revendications 1 à 6, caractérisé en ce qu'il comprend , dans les conditions d'hybridation non stringentes, les étapes suivantes :
- traitement de préhybridation du support (filtre de nitrocellulose ou membrane de nylon), sur lequel est fixé l'acide nucléique, selon l'une quelconque des revendications 1 à 6, à 40°C pendant 6 heures avec une solution ayant la composition suivante : 4 x SSC, 10 x Denhardt ;
- remplacement de la solution de pré-hybridation au contact du support par une solution tampon ayant la composition suivante : 4 x SSC, 1 x Denhardt, 25 mM NaPO₄ pH 7, 2 mM EDTA, 0,5 % SDS, 100 »g/ml de sperme de saumon soniqué, comprenant la sonde nucléotidique marquée notamment de manière radioactive, et préalablement dénaturée par un traitement à 100°C pendant 3 minutes ;
- incubation pendant 12 heures à 40°C,
- lavages successifs avec 2 x SSC à 45°C pendant 15 minutes à deux reprises.

19. Méthode de dépistage, ou de dosage, in vitro d'une ECA ou produit ayant in vivo les propriétés de l'ECA, dans un prélèvement biologique susceptible de le contenir, caractérisée en ce qu'elle comprend :
- la mise en contact d'un anticorps selon la revendication 15, avec le susdit prélèvement biologique dans des conditions permettant la production éventuelle d'un complexe immunologique formé entre l'ECA ou produit qui en est dérivé et l'anticorps sus-mentionné ;
- la détection du susdit complexe immunologique

20. Méthode de dépistage, ou de dosage, in vitro d'un acide nucléique selon l'une quelconque des revendications 1 à 6, dans un prélèvement biologique susceptible de le contenir, caractérisée en ce qu'elle comprend :
- la mise en contact d'une sonde nucléotidique selon la revendication 16, avec le susdit prélèvement biologique dans des conditions permettant la production éventuelle d'un complexe d'hybridation formé entre la sonde et l'acide nucléique sus-mentionnés ;
- la détection du susdit complexe d'hybridation.

21. Méthode de dépistage in vitro selon revendication 19 ou 20, appliquée au diagnostic in vitro de pathologies telles que l'hypertension, de la sarcoïdose et autres maladies granulomateuses, les dysfonctionnements thyroïdiens.

22. Nécessaire ou kit pour la mise en oeuvre d'une méthode de dépistage in vitro selon les revendications 19 et 21 comprenant :
- une quantité déterminée d'un anticorps selon la revendication 15 susceptible de donner lieu à une réaction immunologique avec un polypeptide à détecter selon l'une quelconque des revendications 10 à 14 ;
- avantageusement, un milieu approprié à la formation d'une réaction immunologique entre le polypeptide et l' anticorps sus-mentionnés ;
- avantageusement, des réactifs permettant la détection des complexes immunologiques formés entre le polypeptide et l' anticorps lors de la susdite réaction immunologique.

23. Nécessaire ou kit pour la mise en oeuvre d'une méthode de dépistage in vitro selon la revendication 20 et 21 comprenant :
- une quantité déterminée d'une sonde nucléotidique selon la revendication 16, susceptible de donner lieu à une réaction d'hybridation avec un acide nucléique selon l'une quelconque des revendications 1 à 6 ;
- avantageusement, un milieu approprié à la formation d'une réaction d'hybridation entre l' acide nucléique et la sonde sus-mentionnée ;
- avantageusement, des réactifs permettant la détection des complexes d'hybridation formés entre l'acide nucléique et la sonde lors de la susdite réaction d'hybridation.

24. Méthode de détection ou de dosage, d'un inhibiteur de l'ECA, ou de quantification de son pouvoir inhibiteur, qui comprend la mise en contact d'un polypeptide selon l'une quelconque des revendications 10 à 14 avec ledit inhibiteur, et la détermination du coefficient d'inhibition de l'enzyme par l'inhibiteur, notamment par mesure de l'éventuelle activité enzymatique résiduelle sur un substrat approprié de l'ECA.

25. Compositions pour le traitement de maladies inflammatoires ou infectieuses, notamment de la pancréatite aigue, et des maladies où les kinines jouent une rôle pathogène, caractérisées par l'association d'un polypeptide selon l'une quelconque des revendications 10 à 14, avec un véhicule pharmaceutiquement acceptable.

26. Méthode de dépistage in vitro des polymorphismes du gène codant pour l'ECA au sein d'une population donnée d'individus, réalisé à partir un échantillon biologique tel que le sang prélevé dans ladite population caractérisé en ce qu'il comprend :
- le traitement de l'acide nucléique issu de l'échantillon biologique sus-mentionné réalisé à l'aide d'une enzyme de restriction dans des conditions permettant l'obtention de fragments de restriction issu du clivage dudit acide nucléique au niveau de ces sites de restrictions reconnus par ladite enzyme,
- la mise en contact d'une sonde nucléotidique selon la revendication 16, susceptible de s'hybrider avec les fragments sus-mentionnés dans des conditions permettant la production éventuelle de complexes d'hybridation entre ladite sonde et les fragments de restriction sus-mentionnés,
- la détection des susdits complexes d'hybridation,
- la mesure de taille des éventuels fragments de restriction polymorphes engagés dans les complexes d'hybridation sus-mentionnés.

## Claims

1. Nucleic acid characterized in that it comprises all or part of the nucleic acid sequence of figure 3, and in that it codes for a polypeptide capable of hydrolysing angiotensin I and/or the kinins, in particular bradykinin, or any nucleic acid which differs from the foregoing with respect to its nucleotide sequence only by nucleotide substitutions not leading to the modification of the amino acid sequence of the above-mentioned polypeptide under conditions likely to cause it to lose the above-mentioned properties.

2. Nucleic add according to Claim 1, characterized in that it comprises a DNA sequence delimited by the nucleotides situated at positions 110 and 3940 of Figure 3, coding for mature human ACE.

3. Nucleic acid according to Claim 2, characterized in that said DNA sequence coding for mature human ACE is preceded by a DNA sequence delimited by the nucleotide situated at positions 23 and 109 of Figure 3, this latter coding for a signal peptide of 29 amino acids.

4. Nucleic acid according to Claim 1, characterized in that it comprises a DNA sequence extending between the nucleotide situated between positions 1 and 1177, on the one hand, and the nucleotide situated between positions 3070 and 3940, on the other, of Figure 3.

5. Nucleic acid according to Claim 1, characterized in that it comprises a DNA sequence extending between the nucleotide situated between positions 110 and 1177, on the one hand, and the nucleotide situated between positions 1276 and 1966, on the other, of figure 3.

6. Nucleic add according to Claim 1, characterized in that it comprises a DNA sequence extending between the nucleotide situated between positions 1967 and 2971, on the one hand, and the nucleotide situated between positions 3070 and 3940, on the other, of Figure 3.

7. Nucleic add, characterized in that it comprises a nucleotide sequence according to any one of the Claims 1 to 6 associated with a promoter under the control of which the transcription of said sequence is capable of being performed and, optionally, a DNA sequence coding for transcription termination signals.

8. Recombinant vector, in particular a recombinant plasmid, or recombinant virus, of transforming or infecting a suitable host cell, in particular a eukaryotic cell, said vector containing a DNA fragment according to any one of the Claims 1 to 7 under the control of regulatory elements making possible the expression of this DNA fragment in the host cell, in particular a promoter recognized by the polymerases of said host cell.

9. Procedure for the production of human ACE, mature or not, or a polypeptide derived from human ACE and capable of hydrolysing angiotensin I and/or the kinins, in particular bradykinin, this procedure comprising the transformation of host cells by means of a vector according to Claim 8, the placing of the transformed host cells in culture in a suitable culture medium and the recovery of the enzyme from these cells or the culture medium.

10. Polypeptide encoded by the nucleic acid according to Claim 1, characterized in that it comprises all or part of the peptide sequence delimited by the amino acid situated at positions -29 and 1277 of Figure 3, and in that it is capable of hydrolysing angiotensin I and/or the kinins, in particular bradykinin.

11. Polypeptide according to Claim 10, characterized by the peptide sequence delimited by the amino adds situated at positions 1 and 619 of Figure 3.

12. Polypeptide according to Claim 10, characterized by the peptide sequence delimited by the amino adds situated at positions 620 and 1229 of Figure 3.

13. Polypeptide according to Claim 10, characterized by the peptide sequence delimited by the amino adds situated at positions 350 and 395 of Figure 3.

14. Polypeptide according to Claim 10, characterized by the peptide sequence delimited by the amino acid situated at positions 948 and 993 of Figure 3.

15. Antibody, in particular monoclonal antibody, characterized in that it is directed against a polypeptide according to any one of the Claims 10 to 14.

16. Nucleotide probe characterized in that it is constituted by a nucleotide sequence complementary to all or part of the nucleic acid of Claim 1, or any nucleotide probe differing from the nucleotide sequence of the foregoing only by nucleotide substitutions not leading to the modification of the hybridization properties of said probe with the nucleic acid of Claim 1.

17. Process for the hybridization of a nucleotide probe according to Claim 16 with a nucleic acid according to any one of the Claims 1 to 6, characterized in that it comprises the following steps under stringent conditions of hybridization :
- prehybridization treatment of the support (nitrocellulose filter or nylon membrane) to which is bound the nucleic acid according to any one of the claims 1 to 6 at 65°C for 6 hours with a solution having the following composition : 4 x SSC, 10 x Denhardt;
- replacement of the prehybridization solution in contact with the support by a buffer solution having the following composition : 4 x SSC, 1 x Denhardt, 25 mM NaPO₄ pH 7,2 mM EDTA 0.5% SDS, 100 »g/ml of sonicated salmon sperm DNA containing the nucleotide probe labelled in particular radioactively and previously denatured by treatment at 100°C for 3 minutes;
- incubation for 12 hours at 65°C,
- successive washings with the following solutions :
° 2 x SSC, 1 x Denhardt, 0.5% SDS for 45 minutes at 65°C four times
° 0.2 x SSC, 0.1 x SSC for 45 minutes at 65°C twice,
° 0.1 x SSC 0.1% SDS for 45 minutes at 65°C.

18. Process for the hybridization of a nucleotide probe according to Claim 16 with a nucleic acid according to any one of the Claims 1 to 6, characterized in that it comprises the following steps under non-stringent conditions of hybridization :
- prehybridization treatment of the support (nitrocellulose filter or nylon membrane) to which is bound the nucleic acid according to any one of the Claims 1 to 6 at 40°C for 6 hours with a solution having the following composition: 4 x SSC, 10 x Denhardt;
- replacement of the prehybridization solution in contact with the support by a buffer solution having the following composition : 4 x SSC, 1 x Denhardt, 25 mM NaPO₄ pH 7, 2 mM EDTA 0.5% SDS, 100 »g/ml of sonicated salmon sperm DNA containing the nucleotide probe labelled, in particular radioactively, and previously denatured by treatment at 100°C for 3 minutes;
- incubation for 12 hours at 40°C,
- two successive washings with 2 x SSC at 45°C for 15 minutes.

19. In vitro screening or assay method for an ACE or product having the properties of the ACE in vivo in a biological sample likely to contain it, characterized in that it comprises:
- the placing of an antibody according to Claim 15 in contact with the above-mentioned biological sample under conditions allowing the possible production of an immunological complex formed between the ACE or product which is derived from it and the above-mentioned antibody;
- the detection of the above-mentioned immunological complex.

20. In vitro screening or assay method for a nucleic acid according to any one of the Claims 1 to 6 in a biological sample likely to contain it, characterized in that it comprises :
- the placing of a nucleotide probe according to Claim 16 in contact with the above-mentioned biological sample under conditions leading to the possible production of a hybridization complex formed between the probe and the above-mentioned nucleic acid;
- the detection of the above-mentioned hybridization complex.

21. In vitro screening method according to Claim 19 or 20, applied to the in vitro diagnosis of diseases such as hypertension, sarcoidosis and other granulomatous diseases, thyroid dysfunctioning.

22. Kit for the implementation of an in vitro screening method according to Claims 19 and 21 containing :
- a defined quantity of an antibody according to Claim 15 capable of giving rise to an immunological reaction with a polypeptide to be detected according to any one of the Claims 10 to 14;
- advantageously, a medium suitable for the formation of an immunological reaction between the polypeptide and the antibody mentioned above;
- advantageously, reagents making possible the detection of the immunological complexes formed between the polypeptide and the antibody as a result of the above-mentioned immunological reaction.

23. Kit for the implementation of an in vitro screening method according to Claims 20 and 21 containing :
- a defined quantity of a nucleotide probe according to Claim 16, capable of giving rise to a hybridization reaction with a nucleic acid according to any one of the Claims 1 to 6;
- advantageously, a medium suitable for the formation of a hybridization reaction between the nucleic acid and the probe mentioned above;
- advantageously, reagents making possible the detection of the hybridization complexes formed between the nucleic acid and the probe as a result of the above-mentioned hybridization reaction.

24. Method for the detection or determination of an ACE inhibitor or for the quantification of its inhibitory potency which comprises the placing of a polypeptide according to any one of the Claims 10 to 14 in contact with said inhibitor and the determination of the coefficient of inhibition of the enzyme by the inhibitor, in particular by measurement of possible residual enzymatic activity on a suitable substrate of the ACE.

25. Compositions for the treatment of infectious or inflammatory diseases, in particular of acute pancreatitis, and diseases in which the kinins play a pathogenic role, characterized by the combination of a polypeptide according to any one of the Claims 10 to 14 with a pharmaceutically acceptable vehicle.

26. In vitro screening method for the polymorphisms of the gene coding for the ACE within a given population of individuals, carried out on a biological sample such as blood taken from said population characterized in that it comprises :
- the treatment of the nucleic acid derived from the blological sample mentioned above carried out with the aid of a restriction enzyme under conditions leading to the production of restriction fragments resulting from the cleavage of said nucleic acid at these restriction sites recognized by said enzyme,
- the placing of a nucleotide probe according to Claim 16 in contact with the above-mentioned fragments with which it is capable of hybridizing under conditions leading to the possible production of hybridization complexes between said probe and the above-mentioned restriction fragments,
- the detection of the above-mentioned hybridization complexes,
- the measurement of the size of possible polymorphic restriction fragments participating in the above-mentioned hybridization complexes.

## Patentansprüche

1. Nucleinsäure, dadurch gekennzeichnet, daß sie die ganze oder einen Teil der Nucleotidsequenz der Fig. 3 enthält, und dadurch, daß sie ein Polypeptid codiert, das in der Lage ist, Angiotensin I und/oder die Kinine, insbesondere Bradykinin, zu hydrolysieren, oder jede Nucleinsäure, die sich von der genannten bezüglich ihrer Nucleotidsequenz nur durch Nucleotidsubstitutionen unterscheidet, die keine Änderung der Aminosäuresequenz des genannten Polypeptids in einer Weise zur Folge hat, die geeignet ist, bei diesem den Verlust der genannten Eigenschaften zu verursachen.

2. Nucleinsäure nach Anspruch 1, dadurch gekennzeichnet, daß sie eine DNA-Sequenz enthält, die durch die Nucleotide begrenzt wird, die sich an den Stellen 110 und 3940 der Fig. 3 befinden, die das reife menschliche ACE codiert.

3. Nucleinsäure nach Anspruch 2, dadurch gekennzeichnet, daß der genannten DNA-Sequenz, die das reife menschliche ACE codiert, eine DNA-Sequenz vorangeht, die durch die Nucleotide begrenzt wird, die sich an den Stellen 23 und 109 der Fig. 3 befinden, wobei diese ein Signalpeptid aus 29 Aminosäuren codiert.

4. Nucleinsäure nach Anspruch 1, dadurch gekennzeichnet, daß sie eine DNA-Sequenz enthält, die sich vom an den Stellen 1 bis 1177 der Fig. 3 liegenden Nucleotid bis zum an den Stellen 3070 bis 3940 liegenden Nucleotid erstreckt.

5. Nucleinsäure nach Anspruch 1, dadurch gekennzeichnet, daß sie eine DNA-Sequenz enthält, die sich vom an den Stellen 110 bis 1177 der Fig. 3 liegenden Nucleotid bis zum an den Stellen 1276 bis 1966 liegenden Nucleotid erstreckt.

6. Nucleinsäure nach Anspruch 1, dadurch gekennzeichnet, daß sie eine DNA-Sequenz enthält, die sich vom an den Stellen 1967 bis 2971 der Fig. 3 liegenden Nucleotid bis zum an den Stellen 3070 bis 3940 liegenden Nucleotid erstreckt.

7. Nucleinsäure, dadurch gekennzeichnet, daß sie eine Nucleotidsequenz nach irgendeinem der Ansprüche 1 bis 6 in Verbindung mit einem Promotor, unter dessen Steuerung die Transkription der genannten Sequenz erfolgen kann, und gegebenenfalls eine DNA-Sequenz enthält, die Terminationssignale zur Beendung der Transkription codiert.

8. Rekombinanter Vektor, insbesondere rekombinantes Plasmid oder rekombinanter Virus, der zur Transformation oder Transfektion einer geeigneten Wirtszelle, insbesondere einer eukaryotischen Zelle, in der Lage ist, wobei dieser Vektor ein DNA-Fragment nach irgendeinem der Ansprüche 1 bis 7 gesteuert durch Regelelemente enthält, die die Expression dieses DNA-Fragmentes in der Wirtszelle erlauben, insbesondere durch einen Promotor, der durch die Polymerasen dieser Wirtszelle erkannt wird.

9. Verfahren zur Herstellung des reifen oder nicht reifen menschlichen ACE oder eines aus dem menschlichen ACE entstehenden Polypeptids, das in der Lage ist, Angiotensin I und/oder die Kinine, insbesondere Bradykinin, zu hydrolysieren, wobei dieses Verfahren die Transformation von Wirtszellen mit Hilfe des Vektors nach Anspruch 8 umfaßt, die Kultivierung der transformierten Wirtszellen in einem geeigneten Kulturmedium und die Gewinnung des Enzyms aus diesen Zellen oder dem Kulturmedium.

10. Polypeptid, codiert durch die Nucleinsäure nach Anspruch 1, dadurch gekennzeichnet, daß es die ganze oder einen Teil der Peptidsequenz umfaßt, die durch die Aminosäuren an den Stellen -29 bis 1277 der Fig. 3 begrenzt wird, und dadurch, daß es in der Lage ist, Angiotensin I und/oder die Kinine, insbesondere Bradykinin, zu hydrolysieren.

11. Polypeptid nach Anspruch 10, gekennzeichnet durch die Peptidsequenz, die durch die Aminosäuren an den Stellen 1 und 619 der Fig. 3 begrenzt wird.

12. Polypeptid nach Anspruch 10, gekennzeichnet durch die Peptidsequenz, die durch die Aminosäuren an den Stellen 620 und 1229 der Fig. 3 begrenzt wird.

13. Polypeptid nach Anspruch 10, gekennzeichnet durch die Peptidsequenz, die durch die Aminosäuren an den Stellen 350 und 395 der Fig. 3 begrenzt wird.

14. Polypeptid nach Anspruch 10, gekennzeichnet durch die Peptidsequenz, die durch die Aminosäuren an den Stellen 948 und 993 der Fig. 3 begrenzt wird.

15. Antikörper, insbesondere monoklonaler Antikörper, dadurch gekennzeichnet, daß er gegen ein Polypeptid nach irgendeinem der Ansprüche 10 bis 14 gerichtet ist.

16. Nucleotidsonde, dadurch gekennzeichnet, daß sie aus einer Nucleotidsequenz besteht, die zur gesamten oder einem Teil der Nucleinsäure des Anspruchs 1 komplementär ist, oder jede Nucleotidsonde, die sich von der genannten bezüglich der Nucleotidsequenz nur durch Nucleotidsubstitutionen unterscheidet, die keine Änderungen der Hybridisierungseigenschaften der genannten Sonde mit der Nucleinsäure des Anspruchs 1 zur Folge haben.

17. Verfahren zur Hybridisierung einer Nucleotidsonde nach Anspruch 16 mit einer Nucleinsäure nach irgendeinem der Ansprüche 1 bis 6, dadurch gekennzeichnet, daß es unter stringenten Hybridisierungsbedingungen die folgenden Schritte umfaßt:
- Prähybridisierung der Matrix (Nitrocellulosefilter oder Nylonmembran), auf der die Nucleinsäure nach irgendeinem der Ansprüche 1 bis 6 gebunden ist, bei 65°C für 6 Stunden mit einer Lösung der folgenden Zusammensetzung: 4 x SSC, 10 x Denhardt-Puffer;
- Ersetzen der mit der Matrix in Kontakt stehenden Prähybridisierungslösung durch eine Pufferlösung mit der folgenden Zusammensetzung: 4 x SSC, 1 x Denhardt-Puffer, 25 mM NaPO₄ pH 7, 2 mM EDTA, 0,5% SDS, 100 »g/ml schallbehandeltes Lachssperma, das die, insbesondere radioaktiv, markierte und durch Behandlung bei 100°C für 3 Minuten vorher denaturierte Nucleotidsonde enthält;
- Inkubation für 12 Stunden bei 65°C;
- aufeinanderfolgendes Waschen mit den folgenden Lösungen:
· 2 x SSC, 1 x Denhardt-Puffer, 0,5% SDS viermal für 45 Minuten bei 65°C,
· 0,2 x SSC, 0,1 x SSC zweimal für 45 Minuten bei 65°C,
· 0,1 x SSC, 0,1% SDS für 45 Minuten bei 65°C.

18. Verfahren zur Hybridisierung einer Nucleotidsonde nach Anspruch 16 mit einer Nucleinsäure nach irgendeinem der Ansprüche 1 bis 6, dadurch gekennzeichnet, daß es unter nicht stringenten Hybridisierungsbedingungen die folgenden Schritte umfaßt:
- Prähybridisierung der Matrix (Nitrocellulosefilter oder Nylonmembran), auf der die Nucleinsäure nach irgendeinem der Ansprüche 1 bis 6 gebunden ist, bei 40°C für 6 Stunden mit einer Lösung der folgenden Zusammensetzung: 4 x SSC, 10 x Denhardt-Puffer;
- Ersetzen der mit der Matrix in Kontakt stehenden Prähybridisierungslösung durch eine Pufferlösung mit der folgenden Zusammensetzung: 4 x SSC, 1 x Denhardt-Puffer, 25 mM NaPO₄ pH 7, 2 mM EDTA 0,5% SDS, 100 »g/ml schallbehandeltes Lachssperma, das die, insbesondere radioaktiv, markierte und durch Behandlung bei 100°C für 3 Minuten vorher denaturierte Nucleotidsonde enthält;
- Inkubation für 12 Stunden bei 40°C;
- zwei aufeinanderfolgende Wäschen mit 2 x SSC für 15 Minuten bei 45°C.

19. Methode zum Nachweis oder zur quantitativen Bestimmung in vitro von ACE oder eines Stoffes, der in vivo die Eigenschaften von ACE aufweist, in einer biologischen Probe, die es enthalten kann, dadurch gekennzeichnet, daß sie umfaßt:
- einen Schritt, in dem ein Antikörper nach Anspruch 15 mit der genannten biologischen Probe unter Bedingungen in Berührung gebracht wird, die die eventuelle Erzeugung eines immunologischen Komplexes, bestehend aus ACE oder dem von diesem abgeleiteten Stoff und dem genannten Antikörper, erlauben;
- den Nachweis des genannten immunologischen Komplexes.

20. Methode zum Nachweis oder zur quantitativen Bestimmung in vitro einer Nucleinsäure nach irgendeinem der Ansprüche 1 bis 6 in einer biologischen Probe, die sie enthalten kann, dadurch gekennzeichnet, daß sie umfaßt:
- einen Schritt, in dem eine Nucleotidsonde nach Anspruch 16 mit der genannten biologischen Probe unter Bedingungen in Berührung gebracht wird, die die eventuelle Erzeugung eines Hybridisierungskomplexes, bestehend aus der Sonde und der genannten Nucleinsäure, erlauben;
- den Nachweis des genannten Hybridisierungskomplexes.

21. Methode zum Nachweis in vitro nach Anspruch 19 oder 20, zur Diagnose in vitro von Krankheitserscheinungen wie der Hypertonie, der Sarkoidose und von anderen Granulomatoseerkrankungen sowie von Dysfunktionen der Schilddrüse.

22. Necessaire oder Kit zur Anwendung einer Methode zum Nachweis in vitro nach den Ansprüchen 19 und 21, umfassend:
- eine bestimmte Menge eines Antikörpers nach Anspruch 15, geeignet, um eine immunologische Reaktion mit einem nachzuweisenden Polypeptid nach irgendeinem der Ansprüche 10 bis 14 stattfinden zu lassen;
- vorzugsweise ein geeignetes Medium für eine immunologische Reaktion zwischen diesem Polypeptid und diesem Antikörper;
- vorzugsweise Reagenzien zum Nachweis der immunologischen Komplexe, die in der genannten immunologischen Reaktion aus dem Polypeptid und dem Antikörper gebildet werden.

23. Necessaire oder Kit zur Anwendung einer Methode zum Nachweis in vitro nach den Ansprüchen 20 und 21, umfassend:
- eine bestimmte Menge einer Nucleotidsonde nach Anspruch 16, geeignet, um eine Hybridisierungsreaktion mit einer Nucleinsäure nach irgendeinem der Ansprüche 1 bis 6 stattfinden zu lassen;
- vorzugsweise ein geeignetes Medium für eine Hybridisierungsreaktion zwischen der Nucleinsäure und dieser Sonde;
- vorzugsweise Reagenzien zum Nachweis der Hybridisierungskomplexe, die in der genannten Hybridisierungsreaktion aus der Nucleinsäure und der Sonde gebildet werden.

24. Methode zum Nachweis oder zur quantitativen Bestimmung eines Inhibitors des ACE oder der quantitativen Bestimmung seines Hemmungsvermögens, die einen Schritt umfaßt, in dem ein Polypeptid nach irgendeinem der Ansprüche 10 bis 14 mit dem genannten Inhibitor in Berührung gebracht wird, und die Bestimmung des Koeffizienten der Hemmung des Enzyms durch den Inhibitor, insbesondere durch Messung der eventuell verbleibenden Enzymwirkung an einem geeigneten Substrat des ACE.

25. Zubereitungen zur Behandlung von inflammatorischen und infektiösen Krankheiten, insbesondere der akuten Pankreatitis, und der Krankheiten, bei denen die Kinine eine pathogene Rolle spielen, gekennzeichnet durch die Kombination eine Polypeptids nach irgendeinem der Ansprüche 10 bis 14 mit einem pharmazeutisch angemessenen Träger.

26. Methode zum Nachweis in vitro der Polymorphismen des das ACE codierenden Genes in einer gegebenen Population von Individuen, die aus einer biologischen Probe, wie dem in dieser Population entnommenen Blut, erzeugt wurde, dadurch gekennzeichnet, daß sie umfaßt:
- die Behandlung der aus der genannten biologischen Probe hervorgegangenen Nucleinsäure, die mit Hilfe eines Restriktionsenzyms unter Bedingungen erfolgt, die es erlauben, Restriktionsfragmente zu erhalten, die aus der Spaltung dieser Nucleinsäure an den Schnittstellen entstehen, die vom genannten Enzym erkannt werden;
- einen Schritt, in dem eine Nucleotidsonde nach Anspruch 16, die in der Lage ist, sich mit den genannten Fragmenten zu hybridisieren, mit diesen unter Bedingungen in Berührung gebracht wird, die die eventuelle Erzeugung von Hybridisierungskomplexen aus dieser Sonde und den genannten Restriktionsfragmenten erlauben,
- den Nachweis der genannten Hybridisierungskomplexe,
- die Messung der Länge der eventuellen polymorphen Restriktionsfragmente, die in diesen Hybridisierungskomplexen gebunden sind.
